# EUROPEAN PATENT APPLICATION

(11) **EP 1 749 824 A1**
(43) Date of publication of application: **07.02.2007**
(21) Application number: 05291658.2
(22) Date of filing: 03.08.2005
(51) Int. Cl.: C07D 243/24, C07D 401/04, A61K 31/5513, A61P 25/00

(54) **Benzodiazepine derivatives, their preparation and the therapeutic use thereof**

(71) Applicant: NEURO3D, 68058 Mulhouse Cedex 2 (FR)
(72) Inventor: Abarghaz, Mustapha, 68270 Wittenheim (FR); Biondi, Stefano, San Giovanni Lupatolo, 37057 Verona (IT); Duranton, Jerome, 67000 Strasbourg (FR); Mondadori, Cesare, 4153 Reinach (CH); Wagner, Patrick, 67170 Brumath (FR)
(74) Representative: Tezier Herman, Béatrice

(57) **Abstract**

The invention relates to benzodiazepine derivatives having PDE2 inhibitory activities, as well as therapeutic methods by administering said compounds, in particular for treating various diseases of the central or peripheral nervous system. It further deals with pharmaceutical compositions comprising said compounds and methods for preparing said compounds.

## Description

The invention relates to compounds and their uses, particularly in the pharmaceutical industry. The invention discloses compounds having different interesting biological properties, including PDE2 inhibitory activities, as well as therapeutic methods by administering said compounds, in particular for treating various diseases of the central or peripheral nervous system. It further deals with pharmaceutical compositions comprising said compounds and methods for preparing said compounds.

The compounds of the present invention present a very interesting pharmacological profile, since they are inhibitors of cyclic nucleotide phosphodiesterases and in particular cGS-PDE (cGMP-Stimulated PDEs, type 2-phosphodiesterase, or PDE2).

The intracellular second messenger cAMP or cGMP is broken down and deactivated by phosphodiesterase (PDE), which is classified into at least types I to XI. PDE is widely distributed in the tissue and organs of the body. Among these, type II phosphodiesterase breaks down both cAMP and cGMP and can be activated by cGMP. This type II phosphodiesterase is found in numerous tissues (adipocytes, brain, heart, lungs, kidneys, blood vessels, etc.). PDE2 inhibitors are able to increase or maintain intracellular cAMP and cGMP rates and thereby find therapeutic interests in various pathologies.

The present invention provides compounds having a high inhibiting activity on PDE2, and preferably a selectivity profile with respect to other PDE isoforms. This selectivity profile may extend to other types of enzymes, such as adenosine deaminase. Moreover, compounds of the invention present an interesting effect on the central nervous system (anticonvulsants, anxiolytics, sedative, nootropics, antidepressants) or the peripheral nervous system (against rheumatism, autoinflammatory diseases, against dysfunction of liver due to ageing, diabetes induced pathologies, especially cardiovascular diseases, cancer (angiogenesis, apoptosis), or sepsis). They could also be of interest for treating diseases or disorders due to Trypanosoma (such as sleeping sickness and nagana) and Candida albicans. They also avantageously present no perturbating effect on memory.

The compounds according to the present invention have further been found to block the 5HT transporter. The 5HT transporter regulates serotonergic neurotransmission by mediating the reuptake of 5HT from the synaptic cleft. The more currently prescribed drugs are selective serotonin reuptake inhibitors (SSRIs) which act predominately by inhibiting the reuptake of 5-HT which is released at the synapses and is actively removed from the synaptic cleft via a presynaptic serotonin transport carrier (5-HT-T). SSRIs currently available include citalopram, fluoxetine (Prozac), fluvoxamine, paroxetine (Paxil), and sertraline (Zoloft). Therefore, the compounds according to the present invention are potentially useful for the treatment of depression, anxiety, as well as other serotonin related disorders.

In addition, the compounds according to the present invention also present an agonist activity for the sigma receptor. From studies of the biology and function of sigma receptors, evidence has been presented that sigma receptor ligands may be useful in the treatment of psychosis and movement disorders, such as dystonia and tardive dyskinesia, and motor disturbances associated with Huntington's chorea or Tourette's syndrome and in the treatment of Parkinson's disease (Walker, J. M. et al., Pharmacological Reviews, 1990, 42, 355). The known sigma receptor ligand rimcazole clinically shows effects in the treatment of psychosis (Snyder, S. H., Largent, B. L. J. Neuropsychiatry1989, 1, 7) and a group of sigma receptor ligands have been described to show antihallucinogenic activity in animal models (International Patent Publication No WO 9/03243). Furthermore, sigma receptor ligands have been reported to be involved in modulation of NMDA receptor mediated events in the brain and to act as anti-ischemic agents in in vivo tests (Rao, T. S. et al, Molecular Pharmacology, 1990, 37, 978). In addition to ischemia they may also be useful in the treatment of other such NMDA receptor mediated events, e.g. epilepsy and convulsion. Also, some sigma receptor ligands have been found to show anti-amnesic effects in an animal model (Early et al., Brain Research 1991, 546, 281-286). Sigma ligands have been shown to influence central acetylcholine levels in animal models (Matsuno et al, Brain Research 1992, 575, 315-319; Junien et al, Eur. J. Pharm. 1991, 200, 343-345) and may, therefore, have potential in the treatment of senile dementia of the Alzheimer type. Finally, some guanidine derivatives having sigma receptor activity have been disclosed to be useful as anxiolytics (International Patent Application No. WO 9014067). Therefore, the compounds according to the present invention are potentially useful for these indications.

Accordingly, agents potently acting on the sigma receptors in the central nervous system are believed to be of potential use in the therapy of such conditions.

In view of these different activities, especially towards the nervous system (peripheral and central nervous system), the compounds according to the present invention present a great therapeutic interest.

The present invention discloses therefore compounds having the following general formula (I): 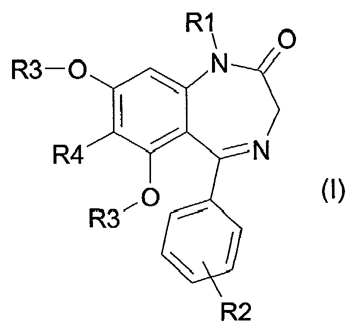 wherein:
- R₁ represents an hydrogen atom, an alkyl, aryl, alkylaryl, or arylalkyl group, wherein said group can be substituted by at least one group preferably selected among the following groups : an alkyl group, a halogen atom, or a halogenoalkyl group, such as for instance trifluoromethyl or difluoromethyl group,
- R₂ represents a hydrogen atom, a halogen atom, an alkyl, an alkoxy, an alkoxyalkyl, an alkoxyalkynyl, an aminoalkyl, a trifluoromethyl, an alkenyl, an alkynyl, an aminoalkynyl, a hydroxy group, CN, CHO, CONH₂ group, or a group of the following formula: - (R₅)ₙNHCOR₆, where R₅ is an alkyl, alkenyl or alkynyl group, n is an integer from 0 to 3, e.g. n is 0, 1, 2 or 3, and R₆ is an alkyl, aryl, aryloxy or alkoxy group,
- R₃, which is the same or different, is a hydrogen atom, an alkyl, halogenoalkyl group, such as for instance trifluoromethyl or difluoromethyl,
- R₄ represents an aryl or heteroaryl group, said aryl or heteroaryl group may be substituted by at least one group preferably selected among the following groups: a halogen atom, alkyl, alkoxy, halogenoalkyl group, such as for instance trifluoromethyl or difluoromethyl,
or a pharmaceutically acceptable salt or solvate thereof.

The present invention also relates to pharmaceutical compositions comprising at least one compound as defined above in a pharmaceutically acceptable vehicle or support, optionally in association with another active agent.

The pharmaceutical composition is more particularly intended to treat diseases associated with abnormal regulation of intracellular cAMP and/or cGMP concentrations.

The present invention also relates to the use of a compound as defined above, for the preparation of a pharmaceutical composition for the treatment of diseases associated with abnormal regulation of intracellular cAMP and/or cGMP concentrations.

The present invention also includes methods of treating diseases associated with dysregulation of intracellular cAMP and/or cGMP concentrations, comprising the administration to a subject in need thereof of an effective amount of a compound as defined above.

Within the context of the present application, the alkyl groups may be linear, cyclic, or branched saturated groups containing from 1 to 10 carbon atoms. Examples of alkyl groups having from 1 to 10 carbon atoms inclusive are methyl, ethyl, propyl, isopropyl, t-butyl, n-butyl, pentyl, hexyl, heptyl, octyl, nonyl, decyl, 2-ethylhexyl, 2-methylbutyl, 2-methylpentyl, 1-methylhexyl, 3-methylheptyl and the other isomeric forms thereof. The cyclic alkyl groups include cyclopropyl, cyclobutyl or cyclopentyl groups. They include also alkyl groups comprising linear, branched, and/or cyclic moieties simultaneously, including a (cycloalkyl)alkyl group, such as for instance cyclopropylmethyl radical. Preferably, the alkyl groups have from 1 to 6 carbon atoms. The alkyl groups can be susbstituted as described below, for instance by an aryl (e.g., arylalkyl), halogen atom (e.g., halogenoalkyl) or alkoxy group (e.g., alkoxyalkyl).

The term alkoxy denotes an alkyl group as defined above attached to the rest of the molecule by an oxygen atom.

The term alkenyl denotes linear or branched groups containing from 2 to 10, preferably 2 to 6, carbon atoms and presenting at least one C=C double bond. Examples of alkenyl groups include in particular the allyl group.

The term alkynyl denotes linear or branched groups containing from 2 to 8 carbon atoms and presenting at least one C≡C triple bond. Examples of alkynyl groups include in particular the ethynyl, propynyl, butynyl, pentynyl, hexynyl group. Such group may be substituted, in particular by amino, alkoxy, NHCOR₆ or aryl as defined below.

The term alkoxyalkynyl, or aminoalkynyl, denotes an alkoxy group as defined above, or respectively an amino group (NH2), attached to the molecule by an alkynyl group as defined above.

The term aminoalkyl stands for a NH2 group attached to the molecule by an alkyl group as defined above.

The term aryl includes any aromatic group comprising from 6 to 18 carbon atoms, preferably from 6 to 14 carbon atoms. Most preferred aryl groups are mono- or bi-cyclic and comprises from 6 to 10 carbon atoms, such as phenyl, α-naphtyl, β-naphtyl.

Another most preferred aryl group is tricyclic and includes antracenyl, or fluorenyl group. When R₄ is an aryl group, it is preferably phenyl, 1-naphtyl, or 2-naphtyl groups.

The term heteroaryl includes any aromatic group comprising from 4 to 18 carbon atoms, preferably from 4 to 14 carbon atoms, and interrupted by one or several heteroatoms selected from N, O, S. Most preferred heteroaryl groups are thienyl, benzothienyl, benzofuryl, pyridyl, pyrimidinyl, pyridazinyl, isoquinolyl, quinolyl, thiazolyl, furyl, pyranyl, pyrrolyl, 2*H*-pyrrolyl, imidazolyl, benzymidazolyl, pyrazolyl, isothiazolyl, isoxazolyl and indolyl groups.

The term arylalkyl group generally stands for an aryl group, preferably phenyl, attached to the molecule by an alkyl group as defined above, such as benzyl or phenethyl. The term alkylaryl group generally stands for an alkyl group attached to the molecule by an aryl group as defined above.

Halogen is understood to refer to fluorine, chlorine, bromine or iodine.

Heteroatom is understood to refer to O, N and S.

According to a particular embodiment, the compounds according to the invention correspond to general formula (I) wherein R₁ represents a hydrogen atom or an alkyl group, preferably a hydrogen atom, a methyl, ethyl, propyl, cyclopropylmethyl group.

According to another embodiment, the compounds according to the invention correspond to general formula (I) wherein R₁ represents an arylalkyl group, in particular a phenylalkyl group, such as benzyl, phenethyl or 3-phenyl-propyl, in which the aryl group may be substituted by a halogenoalkyl group, such as trifluoromethyl.

According to a particular aspect of the invention, the compounds according to the invention correspond to general formula (I), wherein R₂, which is different from hydrogen, is on position 3 of the phenyl group.

According to a particular aspect of the invention, the compounds according to the invention correspond to general formula (I), wherein R₂ represents a hydrogen atom, a halogen atom (preferably Br), CN, or CONH2.

Other preferred compounds according to the invention correspond to compounds of general formula (I), wherein R₂, represents 3-alkoxypropynyl, (preferably 3-methoxypropynyl), 3-aminopropynyl, 3-alkoxypropyl (preferably 3-methoxypropyl), or 3-aminopropyl.

According to another particular aspect of the invention, the compounds according to the invention correspond to general formula (I), wherein R₂ represents a group of the following formula: -(R₅)ₙNHCOR₆ wherein R₅ is an alkyl (preferably propyl) or alkynyl (preferably propynyl) group, n is 1 to 3, and R₆ is an alkoxy (preferably tert-butoxy) group.

In a preferred embodiment, compounds have a formula (I) wherein R₃, which is the same or different, represents an alkyl group, preferably a methyl group.

According to one aspect, the compounds according to the invention correspond to general formula (I) wherein R₄ is a, substituted or not, phenyl group. When the phenyl group is substituted, it is preferably substituted by a halogen atom.

According to another aspect, the compounds of the invention have a general formula (I) wherein R₄ is a, substituted or not, pyridine group. When the pyridine group is substituted, it is preferably substituted by a halogen atom.

When the compounds according to the invention are in the forms of salts, they are preferably pharmaceutically acceptable salts. Such salts include pharmaceutically acceptable acid addition salts, pharmaceutically acceptable base addition salts, pharmaceutically acceptable metal salts, ammonium and alkylated ammonium salts. Acid addition salts include salts of inorganic acids as well as organic acids. Representative examples of suitable inorganic acids include hydrochloric, hydrobromic, hydroiodic, phosphoric, sulfuric, perchloric, and the like. Representative examples of suitable organic acids include formic, acetic, trichloroacetic, trifluoroacetic, propionic, benzoic, cinnamic, citric, fumaric, and the like. Further examples of pharmaceutically acceptable inorganic or organic acid addition salts include the pharmaceutically acceptable salts listed in J. Pharm. Sci. 1977, 66, 2, and in Handbook of Pharmaceutical Salts: Properties, Selection, and Use edited by P. Heinrich Stahl and Camille G. Wermuth 2002. Examples of metal salts include lithium, sodium, potassium, magnesium salts and the like. Examples of ammonium and alkylated ammonium salts include ammonium, methylammonium, dimethylammonium, trimethylammonium, ethylammonium, hydroxyethylammonium, diethylammonium, butylammonium, tetramethylammonium salts and the like. Examples of organic bases include lysine, arginine, guanidine, diethanolamineoline and the like.

Specific examples of compounds of formula (I) which fall within the scope of the present invention include the following compounds:
3-(6,8-Dimethoxy-2-oxo-7-phenyl-2,3-dihydro-1H-benzo[e][1,4]diazepin-5-yl)-benzonitrile
3-[7-(4-Chloro-phenyl)-6,8-dimethoxy-2-oxo-2,3-dihydro-1H-benzo[e][1,4]diazepin-5-yl]-benzonitrile
6,8-Dimethoxy-5,7-diphenyl-1,3-dihydro-benzo[e][1,4]diazepin-2-one
3-(6,8-Dimethoxy-2-oxo-7-pyridin-3-yl-2,3-dihydro-1H-benzo[e][1,4]diazepin-5-yl)-benzonitrile
5-(3-Bromo-phenyl)-6,8-dimethoxy-7-phenyl-1,3-dihydro-benzo[e][1,4]diazepin-2-one
3-(6,8-Dimethoxy-1-methyl-2-oxo-7-phenyl-2,3-dihydro-1H-benzo[e][1,4]diazepin-5-yl)-benzonitrile
3-[7-(4-Chloro-phenyl)-6,8-dimethoxy-1-methyl-2-oxo-2,3-dihydro-1H-benzo[e][1,4]diazepin-5-yl]-benzonitrile
3-(6,8-Dimethoxy-1-methyl-2-oxo-7-pyridin-3-yl-2,3-dihydro-1H-benzo[e][1,4]diazepin-5-yl)-benzonitrile
6,8-Dimethoxy-1-methyl-5,7-diphenyl-1,3-dihydro-benzo[e][1,4]diazepin-2-one
5-(3-Bromo-phenyl)-6,8-dimethoxy-1-methyl-7-phenyl-1,3-dihydro-benzo[e][1,4]diazepin-2-one
3-(6,8-Dimethoxy-2-oxo-7-phenyt-1-ethyl-2,3-dihydro-1H-benzo[e][1,4]diazepin-5-yl)-benzonitrile
3-(6,8-Dimethoxy-2-oxo-7-phenyl-1-propyl-2,3-dihydro-1H-benzo[e][1,4]diazepin-5-yl)-benzonitrile
3-(1-Cyclopropylmethyl-6,8-dimethoxy-2-oxo-7-phenyl-2,3-dihydro-1H-benzo[e][1,4]diazepin-5-yl)-benzonitrile
3-(1-Benzyl-6,8-dimethoxy-2-oxo-7-phenyl-2,3-dihydro-1H-benzo[e][1,4]diazepin-5-yl)-benzonitrile
3-[6,8-Dimethoxy-2-oxo-7-phenyl-1-(4-trifluoromethyl-benzyl)-2,3-dihydro-1H-benzo[e][1,4]diazepin-5-yl]-benzonitrile
3-[6,8-Dimethoxy-2-oxo-7-phenyl-1-(3-phenyl-propyl)-2,3-dihydro-1H-benzo[e][1,4]diazepin-5-yl]-benzonitrile
3-(6,8-Dimethoxy-1-methyl-2-oxo-7-phenyl-2,3-dihydro-1H-benzo[e][1,4]diazepin-5-yl)-benzamide
3-[7-(4-Chloro-phenyl)-6,8-dimethoxy-1-methyl-2-oxo-2,3-dihydro-1H-benzo[e][1,4]diazepin-5-yl]-benzamide
3-(6,8-Dimethoxy-2-oxo-7-pyridin-3-yl-2,3-dihydro-1H-benzo[e][1,4]diazepin-5-yl)-benzamide
3-(6,8-Dimethoxy-2-oxo-7-pyridin-3-yl-2,3-dihydro-1H-benzo[e][1,4]diazepin-5-yl)-benzamide
3-(6,8-Dimethoxy-2-oxo-7-phenyl-1-ethyl-2,3-dihydro-1H-benzo[e][1,4]diazepin-5-yl)-benzamide
3-(6,8-Dimethoxy-2-oxo-7-phenyl-1-propyl-2,3-dihydro-1H-benzo[e][1,4]diazepin-5-yl)-benzamide
3-(1-Cyclopropylmethyl-6,8-dimethoxy-2-oxo-7-phenyl-2,3-dihydro-1H-benzo[e][1,4]diazepin-5-yl)-benzamide
3-(1-Benzyl-6,8-dimethoxy-2-oxo-7-phenyl-2,3-dihydro-1H-benzo[e][1,4]diazepin-5-yl)-benzamide
3-[6,8-Dimethoxy-2-oxo-7-phenyl-1-(4-trifluoromethyl-benzyl)-2,3-dihydro-1H-benzo[e][1,4]diazepin-5-yl]-benzamide
3-[6,8-Dimethoxy-2-oxo-7-phenyl-1-(3-phenyl-propyl)-2,3-dihydro-1H-benzo[e][1,4]diazepin-5-yl]-benzamide
6,8-Dimethoxy-5-[3-(3-methoxy-prop-1-ynyl)-phenyl]-1-methyl-7-phenyl-1,3-dihydro-benzo[e][1,4]diazepin-2-one
{3-[3-(6,8-Dimethoxy-1-methyl-2-oxo-7-phenyl-2,3-dihydro-1H-benzo[e][1,4]diazepin-5-yl)-phenyl]-prop-2-ynyl}-carbamic acid tert-butyl ester
6,8-Dimethoxy-5-[3-(3-methoxy-propyl)-phenyl]-1-methyl-7-phenyl-1,3-dihydro-benzo[e][1,4]diazepin-2-one
{3-[3-(6,8-Dimethoxy-1-methyl-2-oxo-7-phenyl-2,3-dihydro-1H-benzo[e][1,4]diazepin-5-yl)-phenyl]-prop-2-ynyl}-carbamic acid tert-butyl ester
5-[3-(3-Amino-prop-1-ynyl)-phenyl]-6,8-dimethoxy-1-methyl-7-phenyl-1,3-dihydro-benzo[e][1,4]diazepin-2-one
5-[3-(3-Amino-propyl)-phenyl]-6,8-dimethoxy-1-methyl-7-phenyl-1,3-dihydro-benzo[e][1,4]diazepin-2-one

The compounds according to the present invention may be prepared by various methods known to those skilled in the art. Different chemical routes have been carried out and are described below.

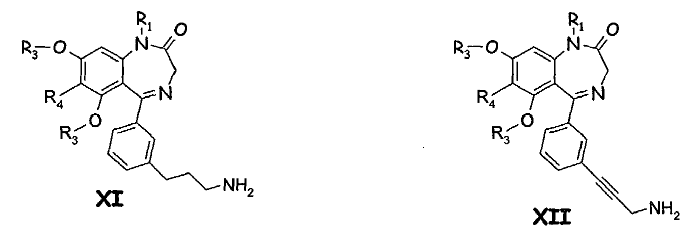

The products of general formula XI and XII can be prepared from compounds of general formula IX or X by hydrolysis of the terminal amino protecting group (=Rx, such as Rx= NHCOOtBu). In particular, when protecting group is tert-butoxycarbonyl, compounds XI can be obtained by acid hydrolysis using protic acids, for example hydrochloric acid or trifluoracetic acid, at a temperature between 0°C and 80°C in an aprotic solvent like dichloromethane, 1,2-dichloroethane, aliphatic or aromatic hydrocarbons. 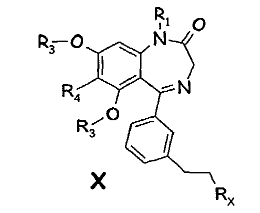 Compounds of general formula X can be prepared by metal catalysed hydrogenation of the compounds of general formula IX using Palladium over carbon in protic solvents such as methanol, ethanol, isopropanol, or butanol at atmospheric pressure. 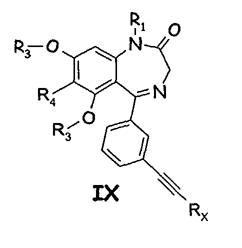 Compounds of general formula **IX** can be prepared by using compounds of general formula **VII,** in which R₁, R_{X} and R₃ are as described above, and substituted alkynes XIII in which Rₓ has the meaning as described above, in a Sonogashira-coupling and nucleophilic substitution with substituted alkynes. Examples of such alkynes include but are not limited to compounds of formula XIII in which R_{X} is an alkyl, arylalkyl, hydroxyalkyl, alkoxyalkyl, aminoalkyl, amidoalkyl and carbamoylalkyl. 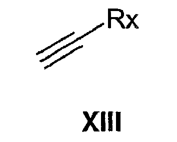 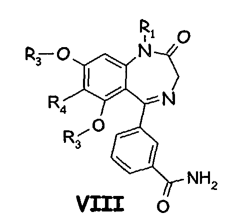 Compound of general formula VIII, in which R₃, R₁ and R₄ have the same meaning as above can be prepared by oxidation with hydrogen peroxide and sodium hydroxide in ethanol at a temperature between 0 and 80°C (Scheme **13**).
Compounds of general formula VIII could also be prepared starting from general formula VII and using a mineral acid such as sulphuric acid in alcohol. 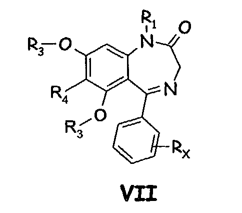 Compounds of general formula **VII,** in which R₃, R_{X} and R₄ are as described above can be obtained by using an alkylating agent of general formula R₁Y, in which R₁ is as described above, and Y can be a suitable leaving group such as a chlorine, bromine, iodine, mesylate and tosylate, in phase transfer conditions. The reaction can be carried out in a suitable solvent such as halogenated hydrocarbons, toluene at room temperature or at boiling point. 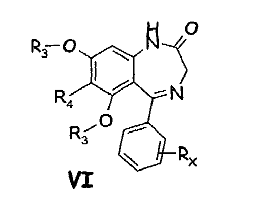 The construction of the benzodiazepinone ring in compounds of general formula **VI** is performed by heating compounds of general formula **IV** and ethyl glycinate hydrochloride in Pyridine at reflux.
An alternative synthesis of compound of general formula **VI** is also performed in two step to give intermediate of general formula **V** that is treated with bromoacetyl bromide and ammonia to yield the cyclic compound of general formula **VI.** 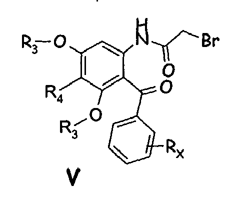 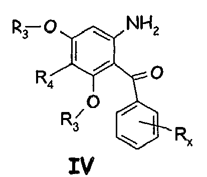 The key intermediates of general formula **IV** can be obtained by a Sugasawa reaction from compounds of general formula **III** using a suitably substituted benzonitrile in a halogenated or aromatic solvent such as dichloromethane, trichloroethylene chlorobenzene, toluene, xylene and most preferably 1,2-dichloroethane with a mixture of Lewis acid such as GaCl₃/BCl₃, InCl₃/BCl₃, FeCl₃/BCl₃, SbCl₅/BCl₃, AgOTf/BCl₃ and most preferably AlCl₃/BCl₃, followed by hydrolysis in HCl. 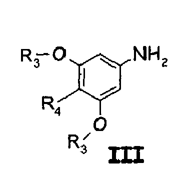 Compounds of general formula **III** can be prepared by using a Palladium catalysed cross-coupling between compounds **II,** in which R₃ and X are as described above, X is an halogen atom, preferably bromine or iodine and boronic acids or esters RB(OR')₂, in which R has the meaning as described above and R' represents H, alkoxy or both R' form with the boron atom and oxygen atoms a 5-membered ring. 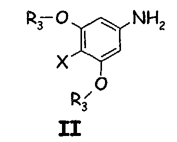 Intermediate of general formula **II**, in which R₃ is CH₃ can be prepared by bromination of the corresponding commercially available 3,5-dimethoxyaniline using a method analogous to that reported in J. Med. Chem. 1989, Vol. 32, N°8, 1936-1942.

It should be understood that other methods of producing these compounds may be designed by the skilled person, based on common general knowledge and following guidance contained in this application.

As indicated above, a further object of this invention relates to a pharmaceutical composition comprising at least one compound of formula (I), as defined above, and a pharmaceutically acceptable vehicle or support.

The compounds may be formulated in various forms, including solid and liquid forms, such as tablets, gel, syrup, powder, aerosol, etc.

The compositions of this invention may contain physiologically acceptable diluents, fillers, lubricants, excipients, solvents, binders, stabilizers, and the like. Diluents that may be used in the compositions include but are not limited to dicalcium phosphate, calcium sulphate, lactose, cellulose, kaolin, mannitol, sodium chloride, dry starch, powdered sugar and for prolonged release tablet-hydroxy propyl methyl cellulose (HPMC). The binders that may be used in the compositions include but are not limited to starch, gelatin and fillers such as sucrose, glucose, dextrose and lactose.
Natural and synthetic gums that may be used in the compositions include but are not limited to sodium alginate, ghatti gum, carboxymethyl cellulose, methyl cellulose, polyvinyl pyrrolidone and veegum. Excipients that may be used in the compositions include but are not limited to microcrystalline cellulose, calcium sulfate, dicalcium phosphate, starch, magnesium stearate, lactose, and sucrose. Stabilizers that may be used include but are not limited to polysaccharides such as acacia, agar, alginic acid, guar gum and tragacanth, amphotsics such as gelatin and synthetic and semi-synthetic polymers such as carbomer resins, cellulose ethers and carboxymethyl chitin.
Solvents that may be used include but are not limited to Ringers solution, water, distilled water, dimethyl sulfoxide to 50% in water, propylene glycol (neat or in water), phosphate buffered saline, balanced salt solution, glycol and other conventional fluids.

The dosages and dosage regimen in which the compounds of formula (I) are administered will vary according to the dosage form, mode of administration, the condition being treated and particulars of the patient being treated. Accordingly, optimal therapeutic concentrations will be best determined at the time and place through experimentation.

The compounds according to the invention can be used enterally. Orally, the compounds according to the invention are suitable administered at the rate of 100 µg to 100 mg per day per kg of body weight. The required dose can be administered in one or more portions. For oral administration, suitable forms are, for example, tablets, gel, aerosols, pills, dragees, syrups, suspensions, emulsions, solutions, powders and granules; a preferred method of administration consists in using a suitable form containing from 1 mg to about 500 mg of active substance.

The compounds according to the invention can also be administered parenterally, for instance in the form of solutions or suspensions for intravenous or intramuscular perfusions or injections. In that case, the compounds according to the invention are generally administered at the rate of about 10 µg to 10 mg per day per kg of body weight; a preferred method of administration consists of using solutions or suspensions containing approximately from 0.01 mg to 1 mg of active substance per ml.

For the compounds of this invention, the dose to be administered, whether a single dose, multiple dose, or a daily dose, will of course vary with the particular compound employed because of the varying potency of the compound, the chosen route of administration, the size of the recipient, the type of disease and the nature of the patient's condition. The dosage to be administered is not subject to definite bounds, but it will usually be an effective amount, or the equivalent on a molar basis of the pharmacologically active free form produced from a dosage formulation upon the metabolic release of the active drug to achieve its desired pharmacological and physiological effects. A doctor skilled in the art for treating the disease will be able to ascertain, without undue experimentation, appropriate protocols for the effective administration of the compounds of this present invention, such as by referring to the earlier published studies on compounds found to have effect on the disease to be treated.

Preferred compounds for use according to the invention include any sub-group or compound as defined above.

Compounds according to the invention may act advantageously on PDE2. Compounds of the invention are preferably selective inhibitors of PDE2, i.e. they present an inhibiting effect on other phosphodiesterases, including for instance PDE3 and PDE4 to a lesser extent. Some compounds present also a specific inhibiting profile for PDE2, including with respect to adenosine deaminase, and present to this respect advantageous therapeutic properties. Most of the compounds of the invention also present a sigma receptor agonist activity and/or a serotonin reuptake inhibition activity.

Compounds of formula (I) are more particularly useful to treat diseases of the central nervous system, especially connected with an abnormal regulation of neurotransmitter effect or a release deficiency of one of the neurotransmitters (e.g. dopamine, noradrenaline, acetylcholine, ...). In particular, they can be used to treat a disease selected in the group consisting of depression, schizophrenia, autism, anxiety, bipolar disorder, attention deficit hyperactivity disorder (ADHD), sleeping disorders, obsessive compulsive disorders (OCD), Post Traumatic Stress Disorder (PTSD), fibromyalgia, Tourette's syndrome, drug or alcohol dependence, epilepsia, movement disorders, such as dystonia and tardive dyskinesia, Alzheimer's disease, Huntington's chorea, Parkinson's disease, amyotrophic lateral sclerosis, multiple sclerosis, obesity, Restless Legs Syndrome (RLS), psychosis, cerebrovascular diseases, migraine, convulsion, amnesia, premenstrual dysphoric disorder (PMDD), post-traumatic stress disorder (PTSD), panic disorders, social disorders, bulimia nervosa and dementia (in particular Lewy body dementia or senile dementia of the Alzheimer type).

The present invention deals also with the use of compounds of the invention, or compositions comprising the same, as anxiolytics, anti-convulsants, nootropics, sedative or to treat memory deficiency or cognitive disorders.

The present invention deals also with the use of compounds of the invention, or compositions comprising the same, for treating rheumatism, autoinflammatory diseases, dysfunction of liver due to ageing, diabetes-induced pathologies, especially cardiovascular diseases, cancer (angiogenesis, apoptosis), or sepsis.

The present invention also relates to the use of compounds of the invention, or compositions comprising the same, for treating disorders due to Trypanosoma (such as sleeping sickness and nagana) or Candida albicans.

According to another aspect, the present invention relates to a method for the treatment of a disease associated with abnormal regulation of intracellular cAMP and/or cGMP rate or to any disease as identified before, comprising administering to a patient in need of such treatment an effective amount of at least one compound of general formula (I) as described above.

Within the context of the invention, the term treatment denotes curative, symptomatic, and preventive treatment. Compounds of the invention can be used in humans with existing disease, including at early or late stages of progression of the disease. The compounds of the invention will not necessarily cure the patient who has the disease but will delay or slow the progression or prevent further progression of the disease, ameliorating thereby the patients' condition, in particular by reducing PDE2 activity, and/or by activating the activity of sigma receptor, and/or by inhibting serotonin reuptake. The compounds of the invention can also be administered to those who do not have the diseases but who would normally develop the disease or be at increased risk for the disease, they will not develop the disease. Treatment also includes delaying the development of the disease in an individual who will ultimately develop the disease or would be at risk for the disease due to age, familial history, genetic or chromosomal abnormalities, and/or due to the presence of one or more biological markers for the disease, such as a known genetic mutation in tissues or fluids. By delaying the onset of the disease, compounds of the invention have prevented the individual from getting the disease during the period in which the individual would normally have gotten the disease or reduce the rate of development of the disease or some of its effects but for the administration of compounds of the invention up to the time the individual ultimately gets the disease. Treatment also includes administration of the compounds of the invention to those individuals thought to be predisposed to the disease. In treating the above diseases, the compounds of the invention are administered in a therapeutically effective amount.

Such compounds, compositions comprising the same, or treatment can be implemented alone or in combination with other active ingredients, compositions or treatments. Moreover, it can correspond to treatment of chronic or acute disorders.

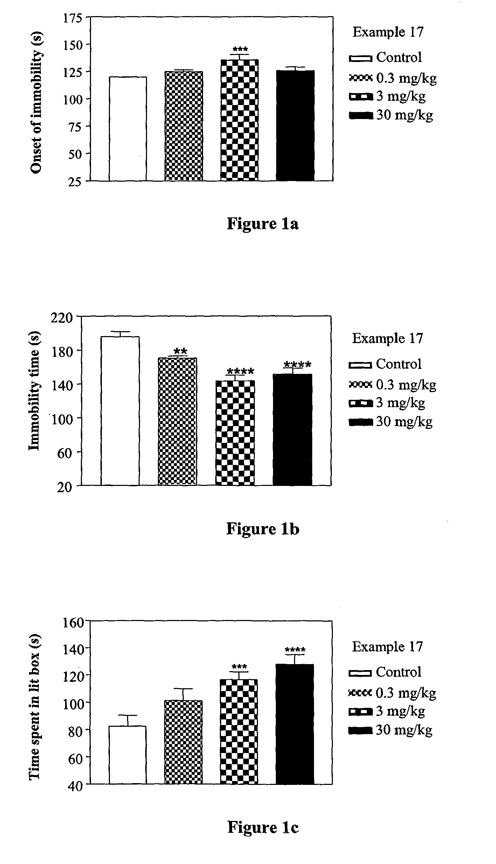

FIGURE 1 represents : Swim test results expressed as mean duration of phases of immobility(s) with different concentrations of a compound according to the invention (figures 1a and 1b) and Light dark test results expressed as time spent in lit box with different concentrations of a compound according to the invention (figure 1c).

Further aspects and advantages of this invention will be disclosed in the following examples, which should be regarded as illustrative and not limiting the scope of this application.

### EXAMPLES

In the Preparations and Examples, unless otherwise stated:
Proton Magnetic Resonance (¹H-NMR) spectra were recorded on Bruker Avance DRX 200, 300 and 400 MHz. Chemical shifts are reported in ppm downfield (d) from Me₄Si, used as internal standard, and are assigned as singlets (s), doublets(d), doublets of doublets (dd), triplets (t), quartets (q) or multiplets (m).
The chromatographic analysis conditions were: column Waters XTerra MS C18 (4.6 x 30mm, 5µm); flow rate 1.0mL/min; mobile phase: aqueous solution of 0,05% TFA (B) and acetonitrile.
The melting point has been performed using a capillary melting point apparatus ref : 7SMP3-0 Bibby.

### 1. PREPARATION OF COMPOUNDS:

### Preparation of intermediates of general formula III (Scheme 1)

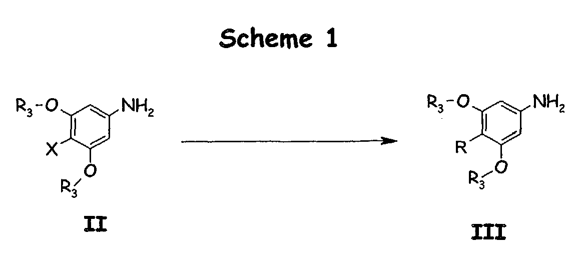

| Intermediate of general formula **III** | X | R₃ | R |
|---|---|---|---|
| 1 | Br | OCH₃ | Ph |
| 2 | Br | OCH₃ | 4-CIPh |
| 3 | Br | OCH₃ | 3-pyridyl |

### Intermediate 1

### 2,6-Dimethoxy-biphenyl-4-ylamine

To 5 mL of degased DMF were added 4-bromo-3,5-dimethoxy-phenylamine Intermediate II (300 mg, 1.29 mmol), benzene boronic acid (400 mg, 3.28 mmol), potassium phosphate (800 mg, 3.55 mmol), Pd(PPh₃)₄ (75 mg, 0.07 mmol). The mixture was stirred for 24 hours at 120°C under nitrogen atmosphere. The working solution was diluted ten times with water and extracted three times with ethyl acetate. The organic phase was dried over Na₂SO₄ and concentrated until dryness. The residue was chromatographed : eluent : AcOEt/Hexane: 1/1. The obtained compound was crystallised from ether/pentane to afford the title compound (235 mg): beige solid, (yield = 79%).
TLC: (AcOEt/Hexane : 1/3) : Rf : 0,7
¹H NMR (CDCl₃, 300 MHz): δ 7.44-7.29 (m, 5H), 6.02 (s, 2H), 3.85 (s, 2H), 3.72 (s, 6H).

### Intermediate 2

### 4'-Chloro-2,6-dimethoxy-biphenyl-4-ylamine

Prepared from 4-bromo-3,5-dimethoxy-phenylamine Intermediate II using the same method described for Intermediate 1 and instead of using benzene boronic acid, we used p-chlorophenyl boronic acid. The title compound (210 mg) was obtained as a white solid, (yield = 62%).
TLC: (AcOEt/Hexane : 1/2): Rf :0,5
¹H NMR (CDCl₃, 300 MHz): δ 7.35-7.25 (m, 4H), 5.99 (s, 2H), 3.78 (s, 2H), 3.75 (s, 6H).

### Intermediate 3

### 3,5-Dimethoxy-4-pyridin-3-yl-phenylamine

Prepared from 4-bromo-3,5-dimethoxy-phenylamine Intermediate II using the same method described for Intermediate 1 and instead of using benzene boronic acid, we used 3-[1,3,2]dioxaborolan-2-yl-pyridine. The title compound (610 mg) was obtained as a beige solid, (yield = 72%).
TLC: (AcOEt/ CH₂Cl₂) : Rf :0,3
¹H NMR (CDCl₃, 200 MHz): δ 8.58-7.56 (m, 1H), 8.47-8.44 (m, 1H), 7.70-7.64 (m, 1H), 7.31-7.24 (m, 1H), 6.01 (s, 2H), 3.70 (s, 2H), 3.75 (s, 6H).

### Preparation of intermediates of general formula IV (Scheme 2)

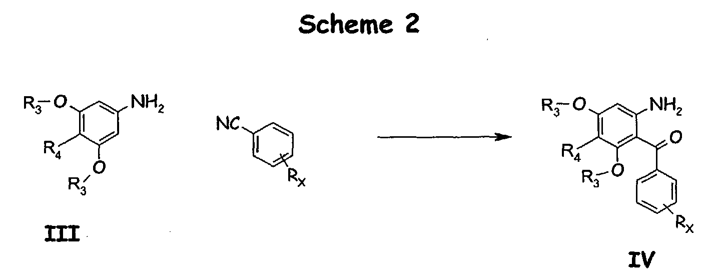

| Intermediate of general formula **IV** | R₃ | R₄ | R_{X} |
|---|---|---|---|
| 4 | OCH₃ | Ph | 3-CNPh |
| 5 | OCH₃ | 4-ClPh | 3-CNPh |
| 6 | OCH₃ | 3-pyridyl | 3-CNPh |
| 7 | OCH₃ | Ph | Ph |
| 8 | OCH₃ | Ph | 3-BrPh |

### Intermediate 4

### 3-(4-Amino-2,6-dimethoxy-biphenyl-3-carbonyl)-benzonitrile

A solution of 2,6-dimethoxy-biphenyl-4-ylamine Intermediate 1 (260 mg, 1.13 mmol), in dichloroethane (1.5 mL) was added dropwise to an ice-cold stirred solution of BCl₃ (1.0 M in CH₂Cl₂, 1.25 mL, 1.25 mmoles) under argon atmosphere.
Then were added isophtalonitrile (218 mg, 1.70 mmol) and anhydrous AlCl₃ (166 mg, 1.25 mmol) and the mixture was stirred at room temperature for 30 min. The mixture was then slowly heated to 60°C and CH₂Cl₂ removed by distillation. Then the solution was refluxed at 78°C for 16 hours. The reaction was allowed to cool to room temperature, treated with aqueous 2N HCl (0.7 mL) and heated at 78°C for 3 hours. Extraction of the mixture with CH₂Cl₂ (2*10 mL) and removal of the solvent afforded the intermediate 1 as a crude mixture. The crude material was chromatographed through silica gel (eluent : CH₂Cl₂ 100% then AcOEt/Hexane : 1/2). The title compound (157 mg) was obtained as a white solid in 39% yield.
TLC: (AcOEt/hexane :1/2): Rf: 0.7
¹H NMR (CDCl₃, 300 MHz): δ 7.99 (s,1H), 7.95-7.93 (m, 1H), 7.77-7.75 (m, 1H), 7.56-7.53 (m, 1H), 7.41-7.30 (m, 5H), 6.16 (s, 1H), 5.47 (s-broad, 2H), 3.81 (s, 3H), 2.88 (s, 3H).

### Intermediate 5

### 3-(4-Amino-4'-chloro-2,6-dimethoxy-biphenyl-3-carbonyl)-benzonitrile

Prepared from 4'-chloro-2,6-dimethoxy-biphenyl-4-ylamine Intermediate 2, using the same method described for Intermediate 4. The title compound (340 mg) was obtained as a yellow solid in 55% yield.
TLC: (AcOEt/hexane :1/2): Rf : 0,6.
¹H NMR (CDCl₃, 300 MHz): δ 7.93-7.88 (m, 2H), 7.76-7.71 (m, 1H), 7.56-7.48 (m, 1H), 7.35-7.22 (m, 4H), 6.09 (s, 1H), 5.57 (s-broad, 2H), 3.78 (s, 3H), 2.88 (s, 3H).

### Intermediate 6

### 3-(6-Amino-2,4-dimethoxy-3-pyridin-3-yl-benzoyl)-benzonitrile

Prepared from 3,5-dimethoxy-4-pyridin-3-yl-phenylamine Intermediate 3, using the same method described for Intermediate 4. The title compound (360 mg) was obtained as a yellow solid in 39% yield.
TLC: (AcOEt/CH₂Cl₂ :4/1): Rf: 0,4.
¹H NMR (CDCl₃, 200 MHz): δ 8.56-8.48 (m, 2H), 7.98-7.90 (m, 2H), 7.77-7.49 (m, 3H), 7.29-7.27 (m, 1H), 6.12 (s, 1H), 5.64 (s-broad, 2H), 3.79 (s, 3H), 2.89 (s, 3H).

### Intermediate 7

### (4-Amino-2,6-dimethoxy-binhenyl-3-yl)-phenyl-methanone

Prepared from 2,6-dimethoxy-biphenyl-4-ylamine Intermediate 1 using the same method described for Intermediate 4 and instead of using isophthalonitrile, we used benzonitrile. The title compound (211 mg) was obtained as a yellow solid, (yield = 45%).
TLC: (AcOEt/Hexane:1/4) : Rf:0,5
¹H NMR (CDCl₃, 300 MHz): δ 7.77-7.74 (m, 2H), 7.51-7.26 (m, 8H), 6.10 (s, 1H), 5.12 (s-broad, 2H), 3.76 (s, 3H), 2.93 (s, 3H).

### Intermediate 8

### (4-Amino-2,6-dimethoxy-biphenyl-3-yl)-(3-bromo-phenyl)-methanone

Prepared from 2,6-dimethoxy-biphenyl-4-ylamine Intermediate 1 using the same method described for Intermediate 4 and instead of using isophtalonitrile, we used 3-bromobenzonitrile. The title compound (1.05 g) was obtained as a yellow solid, (yield = 35%).
TLC: (Hexane/CH₂Cl₂ : 1/4) : Rf :0,5
¹H NMR (CDCl₃, 300 MHz): δ 7.84 (s, 1H), 7.64-7.58 (m, 2H), 7.38-7.25 (m, 6H), 6.15 (s, 1H), 3.77 (s, 3H), 2.90 (s, 3H).

### Preparation of examples of general formula VI (Scheme 3)

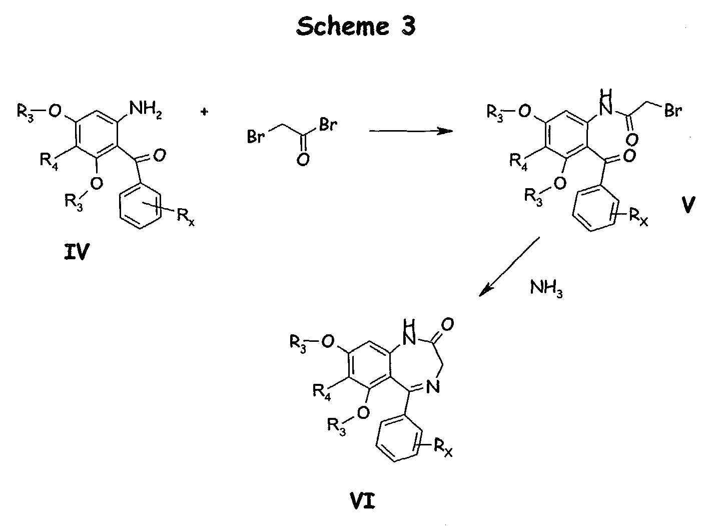

| Examples of general formula VI | R₃ | R₄ | R_{X} |
|---|---|---|---|
| 1 | OCH₃ | Ph | 3-CN |
| 2 | OCH₃ | 4-CIPh | 3-CN |

### Example 1

### 3-(6,8-Dimethoxy-2-oxo-7-phenyl-2,3-dihvdro-1H-benzo[e][1,4]diazepin-5-yl)-benzonitrile

To a solution of 3-(4-amino-2,6-dimethoxy-biphenyl-3-carbonyl)-benzonitrile Intermediate 4 (044 mmol) in methylene chloride (5 mL) at 0-5°C, were added bromoacetyl bromide (0.05 mL, 0.55 mmoles) and dropwise a solution of Na₂CO₃ 10% aq. (0.55 mL). The solution was stirred at this temperature for 30 min. The two layers were separated; the organic layer was washed with water (10 mL), dried over Na₂SO₄, filtered and evaporated under reduced pressure to a crude which was stirred in NH₃ (7N)/MeOH (10 mL) at O°C for 2-4 hours and then refluxed for 16 hours. The working solution was evaporated in vacuum, then triturated in water (30 mL) and filtered. The title compound (116 mg) was obtained as a yellowish solid in 67% yield.
TLC: (AcOEt) : Rf :0,6
¹H NMR (CDCl₃, 300 MHz): δ 8.06 (s-broad, 1H), 7.80-7.77 (m, 2H), 7.56-7.75 (m, 1H), 7.50-7.27 (m, 6H), 6.45 (s, 1H), 4.87-4.84 and 4.05-4.02 (AB system, J = 10 Hz), 3.90 (s, 3H), 2.88 (s, 3H).

### Example 2

### 3-[7-(4-Chloro-phenyl)-6,8-dimethoxy-2-oxo-2,3-dihydro-1H-benzo[e][1,4]diazepin-5-yl]-benzonitrile

Prepared from 3-(4-amino-4'-chloro-2,6-dimethoxy-biphenyl-3-carbonyl)-benzonitrile Intermediate 5 using the same conditions used to prepare Intermediate 1. The title compound (190 mg) was obtained as a yellow solid, (yield = 63%).
TLC: (AcOEt) : Rf :0,5
¹H NMR (CDCl₃, 200 MHz): δ 11.35 (s-broad, 1H), 7.67-7.62 (m, 3H), 7.33-7.27 (m, 5H), 6.69 (s, 1H), 4.09 (s-broad, 2H), 3.81 (s, 3H), 2.50 (s, 3H).

### Preparation of examples of general formula VI (Scheme 4)

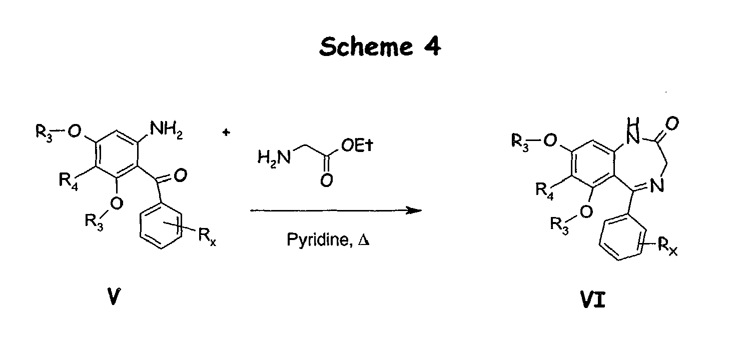

| Example of general formula **VI** | R₃ | R₄ | R_{X} |
|---|---|---|---|
| 3 | OCH₃ | Ph | H |
| 4 | OCH₃ | 3-pyridyl | 3-CN |
| 5 | OCH₃ | Ph | 3-Br |

### Example 3

### 6,8-Dimethoxy-5,7-diphenyl-1,3-dihydro-benzo[e][1,4]diazepin-2-one

A mixture of glycine ethyl ester hydrochloride (177 mg, 1.26 mmol) and Intermediate 1 (211 mg, 0.63 mmol) in dry pyridine (5 mL) was refluxed with stirring for 16 hours. One equivalent of glycine ethyl ester hydrochloride was added at t = 4h, 8h and 24h. Removal of the pyridine under vacuum distillation afforded a tarry residue which was partitioned between ethyl acetate (10 mL) / H₂O (10 mL). The aqueous phase was extracted one time with 10 mL of ethyl acetate; the combined organic phases were dried over Na₂SO₄, filtered and evaporated until dryness. The crude material was chromatographied: eluent: AcOEt/Hexane: 1/1. The title compound was obtained as a white solid (58 mg), yield -: 25%.
¹H NMR (CDCl₃, 200 MHz): δ9.41 (s-broad, 1H), 7.91 (m, 1H), 7.79-7.75 (m, 2H), 7.58-7.50 (m, 1H), 7.41 (s, 1H), 6.67 (s, 1H), 4.36 (s-broad, 2H), 4.00 (s, 3H).

### Example 4

### 3-(6,8-Dimethoxy-2-oxo-7-pyridin-3-yl-2,3-dihydro-1H-benzo[e][1,4]diazepin-5-yl)-benzonitrile

Prepared from 3-(6-amino-2,4-dimethoxy-3-pyridin-3-yl-benzoyl)-benzonitrile Intermediate 6 according to synthesis of Intermediate 3. The title compound (145 mg) was obtained as a yellow solid, yield - 52%.
TLC: (CH₂Cl₂/MeOH : 95/5) : Rf :0,3
1H NMR (CDCl₃, 200 MHz): δ9.09 (s-broad, 1H), 8.61-8.58 (m, 2H), 7.49-7.44 (m, 2H), 7.86-7.67 (m, 4H), 7.50-7.33 (m, 2H), 6.56 (s, 1H), 4.93-4.88 and 4.09-4.04 (AB system, J = 10 Hz), 3.89 (s, 3H), 3.02 (s, 3H).

### Example 5

### 5-(3-Bromo-phenyl)-6,8-dimethoxy-7-phenyl-1,3-dihydro-benzo[e][1,4]diazepin-2-one

Prepared from (4-amino-2,6-dimethoxy-biphenyl-3-yl)-(3-bromo-phenyl)-methanone Intermediate 8 according to synthesis of Example 3. The title compound (680 mg) was obtained as a yellow solid, yield - 62%.
TLC: (AcOEt/CH₂Cl₂ : 1/2) : Rf :0,5
1H NMR (CDCl₃, 300 MHz): δ9.04 (s-broad, 1H), 7.68 (s, 1H), 8.68-8.67 (m, 1H), 7.50-7.30 (m, 6H), 7.23-7.19 (m, 1H), 6.48 (s, 1H), 4.83-4.80 and 4.04-4.01 (AB system, J = 10 Hz), 3.82 (s, 3H), 2.88 (s, 3H).

### Preparation of examples of general formula VII (Scheme 5)

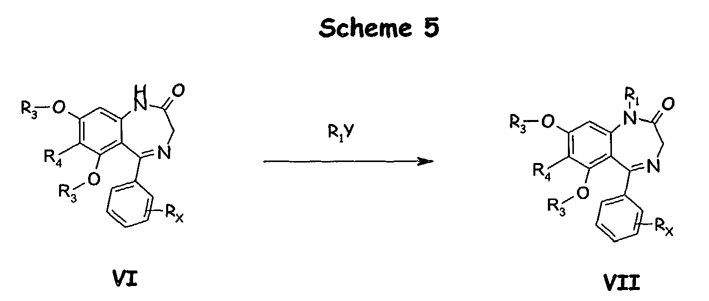

| | | | | |
|---|---|---|---|---|
| Examples of general formula **VII** | R₃ | R₄ | R_{X} | R₁ |
| 6 | OCH₃ | Ph | 3-CN | Me |
| 7 | OCH₃ | 4-ClPh | 3-CN | Me |
| 8 | OCH₃ | 3-pyridyl | 3-CN | Me |
| 9 | OCH₃ | Ph | H | Me |
| 10 | OCH₃ | Ph | 3-Br | Me |
| 11 | OCH₃ | Ph | 3-CN | Et |
| 12 | OCH₃ | Ph | 3-CN | n-Pr |
| 13 | OCH₃ | Ph | 3-CN | 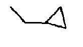 |
| 14 | OCH₃ | Ph | 3-CN | CH₂Ph |
| 15 | OCH₃ | Ph | 3-CN | CH₂(4-CF₃Ph) |
| 16 | OCH₃ | Ph | 3-CN | (CH₂)₃Ph |

### Example 6

### 3-(6,8-Dimethoxy-1-methyl-2-oxo-7-uhenyl-2,3-dihydro-1H-benzo[e][1,4]diazenin-5-yl)-benzonitrile

To a mixture of toluene (30 mL) and Aliquat 336 (30 µL) was introduced methyl iodide (750 µL, 11.58 mmoles) while the mixture was agitated, powdered 3-(6,8-dimethoxy-2-oxo-7-phenyl-2,3-dihydro-1H-benzo[e][1,4]diazepin-5-yl)-benzonitrile Example 1 (2.3 g, 5.8 mmoles) and 50% aqueous sodium hydroxide (10 mL) were added to the reaction mixture. The two-phase system was stirred vigorously for 16 hours. The phases were separated, and the aqueous layer was extracted with ethyl acetate (30 mL). The combined organic extracts were washed with cold water (20 mL); then the organic phase were dried over Na₂SO₄ and concentrated to dryness. The title compound was crystallised from MeOH/Diisopropylether to afford 2.25 g of a white powder, yield -94%.
Rf. (AcOEt/CH₂Cl₂ : 1/4) : 0,6
¹H NMR (CDCl₃, 300 MHz): δ7.91-7.89 (m, 1H), 7.85 (s, 1H), 7.70-7.68 (m, 1H), 7.53-7.30 (m, 6H), 6.70 (s, 1H), 4.91-4.88 and 4.04-4.01 (AB system, J = 11 Hz), 3.89 (s, 3H), 3.49(s, 3H), 2.89 (s, 3H).

### Example 7

### 3-[7-(4-Chloro-phenyl)-6,8-dimethoxy-1-methyl-2-oxo-2,3-dihydro-1H-benzo[e][1,4]diazepin-5-yl]-benzonitrile

Prepared from 3-[7-(4-chloro-phenyl)-6,8-dimethoxy-2-oxo-2,3-dihydro-1H-benzo[e][1,4]diazepin-5-yl]-benzonitrile Example 2 according to synthesis of Example 6. The title compound (75 mg) was obtained as a white solid, yield - 73%.
Rf. (AcOEt/CH₂Cl₂ : 4/1) : 0,7
¹H NMR (CDCl₃, 200 MHz): δ 7.61-7.55 (m, 4H), 7.79-7.75 (m, 2H), 7.37-7.27 (m, 4H), 6.71 (s, 1H), 4.11(s-broad, 2H), 3.89 (s, 3H), 3.84(s, 3H), 2.48 (s, 3H).

### Example 8

### 3-(6,8-Dimethoxy-1-methyl-2-oxo-7-pyridin-3-yl-2,3-dihydro-1H-benzo[e][1,4]diazepin-5-yl)-benzonitrile

Prepared from 3-(6,8-Dimethoxy-2-oxo-7-pyridin-3-yl-2,3-dihydro-1H-benzo[e][1,4]diazepin-5-yl)-benzonitrile Example 4 according to synthesis of Example 6. The title compound (80 mg) was obtained as a beige solid, yield - 86%.
Rf. (AcOEt/CH₂Cl₂ : 4/1) : 0,5
¹H NMR (CDCl₃, 200 MHz): 87.59-7.55 (m, 2H), 7.89-7.83 (m, 2H), 7.70-7.65 (m, 2H), 7.53-7.49 (m, 1H), 7.38-7.31 (m, 1H), 6.69 (s, 1H), 4.91-4.86 and 4.01-3.96 (AB system, J = 10 Hz), 3.88 (s, 3H), 3.46 (s, 3H), 2.95 (s, 3H).

### Example 9

### 6,8-Dimethoxy-1-methyl-5,7-diuhenyl-1,3-dihydro-benzo[e][1,4]diazenin-2-one

Prepared from 6,8-dimethoxy-5,7-diphenyl-1,3-dihydro-benzo[e][1,4]diazepin-2-one Example 3 according to synthesis of Example 6. The title compound ( 27 mg) was obtained as a beige solid, yield - 57%.
Rf. (AcOEt/Hexane : 4/1) : 0,4
¹H NMR (CDCl₃, 400 MHz): δ 7.57-7.55 (m, 2H), 7.54-7.28 (m, 8H), 6.64 (s, 1H), 4.84-4.80 and 4.01-3.98 (AB system, J = 12 Hz), 3.84 (s, 3H), 3.44(s, 3H), 2.85 (s, 3H).

### Example 10

### 5-(3-Bromo-phenyl)-6,8-dimethoxy-1-methyl-7-phenyl-1,3-dihydro-benzo[e][1,4]diazepin-2-one

Prepared from 5-(3-bromo-phenyl)-6,8-dimethoxy-7-phenyl-1,3-dihydro-benzo[e][1,4]diazepin-2-one Example 5 according to synthesis of Example 6. The title compound (590 mg) was obtained as a beige solid, yield - 92%.
Rf. (AcOEt/Hexane : 4/1) : 0,4
¹H NMR (CDCl₃, 400 MHz): δ 7.70-7.69 (m, 1H), 7.51-7.49 (m, 2H), 7.40-7.32 (m, 5H), 7.25-7.21 (m, 1H), 6.64 (s, 1H), 4.84-4.82 and 3.98-69 (AB system, J = 11 Hz), 3.85 (s, 3H), 3.45(s, 3H), 2.87 (s, 3H).

### Example 11

### '3-(6,8-Dimethoxy-2-oxo-7-phenyl-1-ethyl-2,3-dihydro-1H-benzo[e][1,4]diazepin-5-yl)-benzonitrile

Prepared from 3-(6,8-dimethoxy-2-oxo-7-phenyl-2,3-dihydro-1H-benzo[e][1,4]diazepin-5-yl)-benzonitrile Example 1 according to synthesis of Example 6 and instead of using methyl iodide, we used ethyl iodide. The title compound (105 mg) was obtained as a beige solid, yield - 76%.
Rf. (AcOEt/CH₂Cl₂ : 1/1) : 0,7
¹H NMR (CDCl₃, 400 MHz): δ 7.89-7.85 (m, 2H), 7.70-7.68 (m, 1H), 7.53-7.50 (m, 1H), 7.44-7.30 (m, 5H), 6.77 (s, 1H), 4.88-4.85 and 4.03-4.01 (AB system, J = 11 Hz), 4.39-4.32 (m, 1H), 3.88 (s, 3H), 3.83-3.78 (m, 1H), 2.87 (s, 3H) 1.25-1.22 (m, 3H).

### Example 12

### '3-(6,8-Dimethoxy-2-oxo-7-phenyl-1-proenyl-2,3-dihydro-1H-benzo[e][1,4]diazepin-5-yl)-benzonitrile

Prepared from 3-(6,8-dimethoxy-2-oxo-7-phenyl-2,3-dihydro-1H-benzo[e][1,4]diazepin-5-yl)-benzonitrile Example 1 according to synthesis of Example 6 and instead of using methyl iodide, we used propyl iodide. The title compound (105 mg) was obtained as a beige solid, yield - 76%.
Rf. (AcOEt/CH₂Cl₂ : 1/1) : 0,7
¹H NMR (CDCl₃, 400 MHz): δ 7.89-7.89 (m, 1H), 6.75 (s, 1H), 7.82 (s, 1H), 7.71-7.59 (m, 1H), 7.53-7.34 (m, 5H), 6.76 (s, 1H), 4.87-4.85 and 4.01-3.99 (AB system, J = 10 Hz), 3.87 (s, 3H), 2.88 (s, 3H).

### Example 13

### '3-(1-Cyclopropylmethyl-6,8-dimethoxy-2-oxo-7-nhenyl-2,3-dihydro-1H-benzo[e][1,4]diazepin-5-yl)-benzonitrile

Prepared from 3-(6,8-dimethoxy-2-oxo-7-phenyl-2,3-dihydro-1H-benzo[e][1,4]diazepin-5-yl)-benzonitrile Example 1 according to synthesis of Example 6 and instead of using methyl iodide, we used bromomethyl-cyclopropane. The title compound (85 mg) was obtained as a beige solid, yield - 60%.
Rf. (AcOEt/CH₂Cl₂ : 3/2) : 0,6
¹H NMR (CDCl₃, 400 MHz): δ 7.87-7.78 (m, 2H), 7.86-7.84 (m, 1H), 7.68-7.66 (m, 1H), 7.41-7.34 (m, 5H), 6.81 (s, 1H), 4.86-4.83 and 4.02-3.99 (AB system, J = 11 Hz), 4.11-4.08 (m, 1H), 3.86 (s, 3H), 3.75-3.70 (m, 1H), 2.85 (s, 3H), 1.09-1.04 (m, 1H), 0.50-0.48-3.70 (m, 1H), 0.33-0.32 (m, 1H), 0.30-0.25 (m, 2H), 0.17-0.15 (m, 1H).

### Example 14

### '3-(1-Benzyl-6,8-dimethoxy-2-oxo-7-phenyl-2,3-dihydro-1H-benzo[e][1,4]diazenin-5-yl)-benzonitrile

Prepared from 3-(6,8-dimethoxy-2-oxo-7-phenyl-2,3-dihydro-1H-benzo[e][1,4]diazepin-5-yl)-benzonitrile Example 1 according to synthesis of Example 6 and instead of using methyl iodide, we used bromomethyl-benzene. The title compound (95 mg) was obtained as a white solid, yield - 62%.
Rf. (AcOEt/CH₂Cl₂: 2/1) : 0,7
¹H NMR (CDCl₃, 400 MHz): δ 7.70-7.63 (m, 2H), 7.46-7.35 (m, 5H), 7.33-7.18 (m, 5H), 7.12-7.10 (m, 2H), 6.74 (s, 1H), 5.60-5.57 and 4.86-4.83 (AB system, J = 12 Hz), 4.95-4.93 and 4.10-4.08 (AB system, J = 9 Hz), 3.72 (s, 3H), 2.77 (s, 3H).

### Example 15

### '3-[6,8-Dimethoxy-2-oxo-7-Dhenyl-1-(4-trifluoromethyl-benzyl)-2.3-dihydro-1H-benzo[e] [1,4] diazepin-5-yl]-benzonitrile

Prepared from 3-(6,8-dimethoxy-2-oxo-7-phenyl-2,3-dihydro-1H-benzo[e][1,4]diazepin-5-yl)-benzonitrile Example 1 according to synthesis of Example 6 and instead of using methyl iodide, we used 1-bromomethyl-4-trifluoromethyl-benzene. The title compound (120 mg) was obtained as a white solid, yield - 69%.
Rf. (AcOEt/CH₂Cl₂ : 1/4) : 0,7
¹H NMR (CDCl₃, 400 MHz): δ 7.73 (s, 1H), 7.68-7.66 (m, 1H), 7.53-7.51 (m, 1H), 7.46-7.33 (m, 5H), 7.30-7.23 (m, 5H), 6.68 (s, 1H), 5.59-5.55 and 4.97-4.93 (AB system, J = 15 Hz), 4.97-4.94 and 4.13-4.10 (AB system, J = 11 Hz), 4.11-4.08 (m, 1H), 3.72 (s, 3H), 2.79 (s, 3H).

### Example 16

### '3-[6,8-Dimethoxy-2-oxo-7-phenyl-1-(3-phenyl-propyl)-2,3-dihydro-1H-benzo[e][1,4]diazepin-5-yl]-benzonitrile

Prepared from 3-(6,8-dimethoxy-2-oxo-7-phenyl-2,3-dihydro-1H-benzo[e][1,4]diazepin-5-yl)-benzonitrile Example 1 according to synthesis of Example 6 and instead of using methyl iodide, we used (3-bromo-propyl)-benzene. The title compound (100 mg) was obtained as a white solid, yield - 62%.
Rf. (AcOEt/CH₂Cl₂ : 2/1) : 0,7
¹H NMR (CDCl₃, 400 MHz): δ 7.79-7.77 (m, 1H), 7.75 (s, 1H), 7.59-7.57 (m, 1H), 7.38-7.31 (m, 5H), 7.25-7.18 (m, 4H), 7.06-7.04 (m, 2H), 6.61 (s, 1H), 4.89-4.86 and 4.02-3.99 (AB system, J = 12 Hz), 4.45-4.40 (m, 1H), 3.73 (s, 3H), 2.79 (s, 3H), 3.73-3.69 (m, 1H), 4.56-4.53 (m, 2H), 2.05-2.01 (m, 1H), 1.88-1.83 (m, 1H).

### Preparation of examples of general formula VIII (Scheme 6)

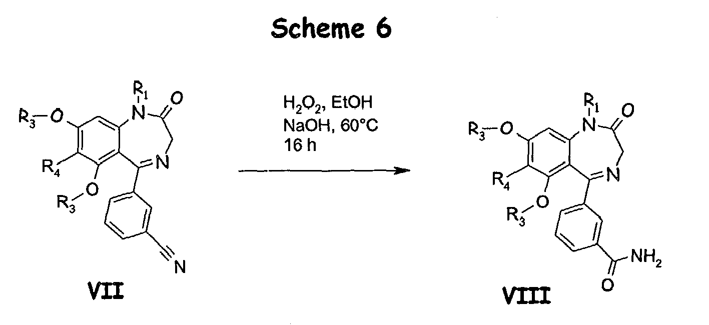

| Examples of general formula **VIII** | R₃ | R₄ | R₁ |
|---|---|---|---|
| 17 | OCH₃ | Ph | Me |
| 18 | OCH₃ | 4-ClPh | Me |
| 19 | OCH₃ | 3-pyridyl | H |
| 20 | OCH₃ | 3-pyridyl | Me |
| 21 | OCH₃ | Ph | Et |
| 22 | OCH₃ | Ph | n-Pr |
| 23 | OCH₃ | Ph | 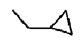 |
| 24 | OCH₃ | Ph | CH₂Ph |
| 25 | OCH₃ | Ph | CH₂(4-CF₃Ph) |
| 26 | OCH₃ | Ph | (CH₂)₃Ph |

### Example 17

### '3-(6,8-Dimethoxy-1-methyl-2-oxo-7-phenyl-2,3-dihydro-1H-benzo[e][1,4]diazepin-5-yl)-benzamide

To a solution of compound 3-(6,8-dimethoxy-1-methyl-2-oxo-7-phenyl-2,3-dihydro-1H-benzo[e][1,4]diazepin-5-yl)-benzonitrile Example 6 (90 mg, 0.22 mmol) in absolute ethanol (2 ml), were added dropwise H₂O₂ aqueous (30% wt in water, 110 □1), followed by aqueous NaOH (0.5 M, 40 µl). The solution was stirred at RT for 16 hours. Removal of ethanol in vacuum gave the crude material which was purified by silica gel column chromatography with CH₂Cl₂/MeOH: 95/5 to give after trituration from ether title compound (35 mg) as a white solid, yield - 37%.
Rf. (AcOEt) : 0,1.
¹H NMR (CDCl₃, 400 MHz): δ 8.09 (m, 1H), 7.89-7.87 (m, 1H), 7.78-7.76 (m, 1H), 7.50-7.46 (m, 1H), 7.42-7.34 (m, 5H), 6.68 (s, 1H), 6.29 and 5.67 (large s, 2H), 4.83-4.77 and 3.84-3.78 (AB system, J = 11 Hz, 2H), 3.93 (s, 3H), 4.88-4.86 and 4.03-4.01 (AB system, J =10 Hz), 3.88 (s, 3H), 3.49 (s, 3H), 2.84 (s, 3H).

### Example 18

### '3-[7-(4-Chloro-phenyl)-6,8-dimethoxy-1-methyl-2-oxo-2,3-dihydro-1H-benzo[e][1.4]diazepin-5-yl]-benzamide

Prepared from 3-[7-(4-chloro-phenyl)-6,8-dimethoxy-1-methyl-2-oxo-2,3-dihydro-1H-benzo[e][1,4]diazepin-5-yl]-benzonitrile Example 7 according to synthesis of Example 17. The title compound (28 mg) was obtained as a yellow solid, yield - 77%.
Rf. (AcOEt) : 0,1.
¹H NMR (CDCl₃, 200 MHz): δ 7.86-7.82 (m, 2H), 7.55-7.52 (m, 2H), 7.35-7.27 (m, 4H), 6.70 (s, 1H), 6.10 and 5.70 (large s, 2H), 4.11(large s, 2H), 3.89 (s, 3H), 3.84 (s, 3H), 2.48 (s, 3H).

### Example 19

### '3-(6,8-Dimethoxy-2-oxo-7-pyridin-3-yl-2,3-dihydro-1H-benzo[e][1,4]diazepin-5-yl)-benzamide

Prepared from 3-[7-(4-chloro-phenyl)-6,8-dimethoxy-1-methyl-2-oxo-2,3-dihydro-1H-benzo[e][1,4]diazepin-5-yl]-benzonitrile Example 4 according to synthesis of Example 17. The title compound (28 mg) was obtained as a yellow solid, yield - 70%.
Rf. (AcOEt) : 0,1.
¹H NMR (CDCl₃, 200 MHz): δ 9.33 (s, 1H), 8.54 (s, 2H), 7.99 (s, 1H), 7.87-7.84 (m, 1H), 7.72-7.63 (m, 2H), 7.47-7.43 (m, 1H), 7.33-7.26 (m, 1H), 6.48 (s, 1H), 6.47 and 6.06 (large s, 2H), 4.84-4.79 and 4.05-3.99 (AB system, J = 11 Hz), 3.75 (s, 3H), 2.94 (s, 3H).

### Example 20

### '3-(6,8-Dimethoxy-2-oxo-7-pyridin-3-yl-2,3-dihydro-1H-benzo[e][1,4]diazepin-5-yl)-benzamide

Prepared from 3-(6,8-dimethoxy-1-methyl-2-oxo-7-pyridin-3-yl-2,3-dihydro-1H-benzo[e][1,4]diazepin-5-yl)-benzonitrile Example 9 according to synthesis of Example 17. The title compound (25 mg) was obtained as a yellow solid, yield - 68%.
¹H NMR (CDCl₃, 200 MHz): δ 8.58-8.54 (m, 2H), 8.05 (s, 1H), 7.86-7.66 (m, 3H), 7.49-7.29 (m, 2H), 6.67 (s, 1H), 6.25 and 5.70 (large s, 2H), 4.88-4.83 and 4.03-3.98 (AB system, J = 10 Hz), 3.87 (s, 3H), 3.45 (s, 3H), 2.91 (s, 3H).

### Example 21

### "3-(6,8-Dimethoxy-2-oxo-7-phenyl-1-ethyl-2,3-dihydro-1H-benzo[e][1,4]diazepin-5-yl)-benzamide

Prepared from 3-(6,8-dimethoxy-2-oxo-7-phenyl-1-ethyl-2,3-dihydro-1H-benzo[e][1,4]diazepin-5-yl)-benzonitrile Example 11 according to synthesis of Example 17. The title compound (65 mg) was obtained as a white solid, yield - 89%.
¹H NMR (CDCl₃, 400 MHz): δ 8.12 (s, 1H), 7.90-7.88 (m, 1H), 7.71-7.69 (m, 1H), 7.49-7.36 (m, 6H), 6.76 (s, 1H), 6.27 and 5.64 (large s, 2H), 4.85-4.83 and 4.03-4.01 (AB system, J = 10 Hz), 4.38-4.34 (m, 1H), 3.87 (s, 3H), 3.84-3.79 (m, 1H), 2.81 (s, 3H) 1.27-1.23 (m, 3H).

### Example 22

### '3-(6,8-Dimethoxy-2-oxo-7-nhenyl-1-prouyl-2,3-dihydro-1H-benzo[e][1,4]diazepin-5-yl)-benzamide

Prepared from '3-(6,8-dimethoxy-2-oxo-7-phenyl-1-propyl-2,3-dihydro-1H-benzo[e][1,4]diazepin-5-yl)-benzonitrile Example 12 according to synthesis of Example 17. The title compound (70 mg) was obtained as a white solid, yield - 93%.
¹H NMR (CDCl₃, 400 MHz): δ 8.07 (s, 1H), 7.88-7.87 (m, 1H), 7.86-7.85 (m, 1H), 7.69-7.32 (m, 6H), 6.72 (s, 1H), 6.22 and 5.74 (large s, 2H), 4.82-4.80 and 4.00-3.98 (AB system, J = 10Hz), 4.38-4.33 (m, 1H), 3.85 (s, 3H), 3.66-3.60 (m, 1H), 2.87 (s, 3H), 1.69-1.64 (m, 1H), 1.57-1.52 (m, 1H), 0.82-0.78 (m, 3H).

### Example 23

### '3-(1-Cyclopronylmethyl-6,8-dimethoxy-2-oxo-7-phenyl-2,3-dihydro-1H-benzo[e][1,4]diazepin-5-yl)-benzamide

Prepared from '3-(1-cyclopropylmethyl-6,8-dimethoxy-2-oxo-7-phenyl-2,3-dihydro-1H-benzo[e][1,4]diazepin-5-yl)-benzonitrile Example 13 according to synthesis of Example 17. The title compound (60 mg) was obtained as a white solid, yield - 97%.
¹H NMR (CDCl₃, 400 MHz): δ 8.08 (s, 1H), 7.87-7.86 (m, 1H), 7.85-7.84 (m, 1H), 7.71-7.33 (m, 6H), 6.79 (s, 1H), 6.19 and 5.65 (large s, 2H), 4.83-4.80 and 4.02-3.99 (AB system, J = 11 Hz), 4.12-4.06 (m, 1H), 3.84 (s, 3H), 3.74-3.69 (m, 1H), 2.79 (s, 3H), 1.10-1.07(m, 1H), 0.49-0.47 (m, 1H), 0.36-0.33 (m, 1H), 0.27-0.25 (m, 1H), 0.17-0.15 (m, 1H),.

### Example 24

### '3-(1-Benzyl-6,8-dimethoxy-2-oxo-7-phenyl-2,3-dihydro-1H-benzo[e][1,4]diazepin-5-yl)-benzamide

Prepared from '3-(1-benzyl-6,8-dimethoxy-2-oxo-7-phenyl-2,3-dihydro-1H-benzo[e][1,4]diazepin-5-yl)-benzonitrile Example 14 according to synthesis of Example 17. The title compound (60 mg) was obtained as a white solid, yield - 90%.
¹H NMR (CDCl₃, 400 MHz): δ 7.90-7.88 (m, 1H), 7.81 (s, 1H), 7.52-7.50 (m, 1H), 7.71-7.33 (m, 6H), 7.21-7.16 (m, 5H), 6.70 (s, 1H), 6.09 and 5.63 (large s, 2H), 5.54-50 and 4.12-4.09 (AB system, J = 12 Hz), 4.92-4.90 (m, 2H), 3.68 (s, 3H), 2.74 (s, 3H).

### Example 25

### '3-[6,8-Dimethoxy-2-oxo-7-Dhenyl-1-[4-trifluoromethyl-benzyl)-2,3-dihydro-1H-benzo[e][1,4]diazepin-5-yl]-benzamide

Prepared from '3-[6,8-dimethoxy-2-oxo-7-phenyl-1-(4-trifluoromethyl-benzyl)-2,3-dihydro-1H-benzo[e][1,4]diazepin-5-yl]-benzonitrile Example 15 according to synthesis of Example 17. The title compound (75 mg) was obtained as a white solid, yield - 97%.
¹H NMR (CDCl₃, 400 MHz): δ 7.95 (s, 1H), 7.88-7.86 (m, 1H), 7.46-7.35 (m, 5H), 7.30-7.25 (m, 6H), 6.66 (s, 1H), 6.13 and 5.65 (large s, 2H), 5.60-56 and 4.97-4.94 (AB system, J = 13 Hz), 4.93-4.91-4.13-4.11 (AB system, J = 10 Hz), 3.70 (s, 3H), 2.74 (s, 3H).

### Example 26

### '3-[6,8-Dimethoxy-2-oxo-7-phenyl-1-(3-phenyl-propyr)-2,3-dihydro-1H-benzo[e][1,4]diazepin-5-yl]-benzamide

Prepared from '3-[6,8-dimethoxy-2-oxo-7-phenyl-1-(3-phenyl-propyl)-2,3-dihydro-1H-benzo[e][1,4]diazepin-5-yl]-benzonitrile Example 16 according to synthesis of Example 17. The title compound (50 mg) was obtained as a white solid, yield - 71 %.
¹H NMR (CDCl₃, 400 MHz): δ 8.07 (s, 1H), 7.90-7.88 (m, 1H), 7.74-7.73 (m, 1H), 7.48-7.67 (m, 6H), 7.24-7.18 (m, 3H), 7.05-7.04 (m, 2H), 6.62 (s, 1H), 6.09 and 5.59 (large s, 2H), 4.87-4.85 and 4.04-4.02 (AB system, J = 12 Hz), 4.47-4.43 (m, 1H), 3.74 (s, 3H), 3.74-3.72 (m, 1H), 2.82 (s, 3H), 2.57-2.52 (m, 2H), 2.08-2.05 (m, 1H), 1.89-1.86 (m, 1H).

### Preparation of Examples of general formula IX (Scheme 7)

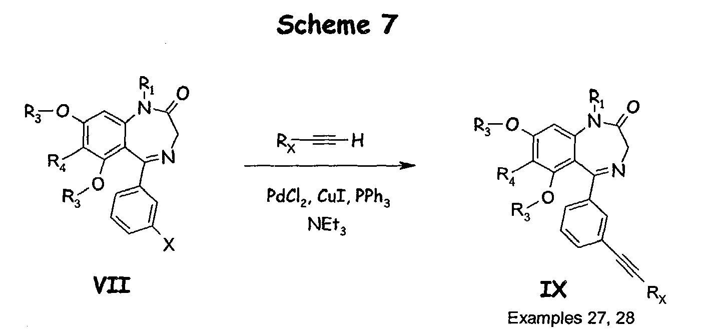

| Examples of general formula **IX** | R₃ | R₄ | R₁ | Rx |
|---|---|---|---|---|
| 27 | OCH₃ | Ph | Me | CH₂OMe |
| 28 | OCH₃ | Ph | Me | CH₂NHBoc |

### Example 27

### '6,8-Dimethoxy-5-[3-(3-methoxy-prop-1-ynyl)-phenyl]-1-methyl-7-phenyl-1,3-dihydro-benzo[e][1,4]diazepin-2-one

To 5 mL of degazed acetonitrile were 5-(3-bromo-phenyl)-6,8-dimethoxy-7-phenyl-1,3-dihydro-benzo[e][1,4]diazepin-2-one Example 10 (150 mg, 0.32 mmol), 3-methoxy-propyne (250 □L, 0.96mmol), copper iodide (3 mg, 0.016 mmol), (PPh₃)₂PdCl₂ (23 mg, 0.032 mmol) and triethylamine (0.5 mL). The mixture was stirred for 16 hours at 60°C under nitrogen atmosphere. The working solution was evaporated under vacuum. The residue was partitioned from water and ethyl acetate and extracted two more times with ethyl acetate. The organic phase was dried over Na₂SO₄ and concentrated until dryness. The residue was chromatographied : (eluent AcOEt/CH₂Cl₂ : 1/2). The title compound (85 mg) was obtained as a beige solid, yield 58- %.
Rf. (AcOEt/CH₂Cl₂ : 1/2) : 0,4
¹H NMR (CDCl₃, 400 MHz): δ 7.59-7.56 (m, 2H), 7.46-7.44 (m, 1H), 7.41-7.38 (m, 2H), 7.34-7.31 (m, 4H), 6.64 (s, 1H), 4.84-4.81 and 3.99-3.96 (AB system, J = 12 Hz), 4.30 (s, 2H), 3.85 (s, 3H), 3.45 (s, 3H), 3.44 (s, 3H), 2.84 (s, 3H).

### Example 28

### {3-[3-(6,8-Dimethoxy-1-methyl-2-oxo-7-phenyl-2,3-dihydro-1H-benzo[e][1.4]diazepin-5-yl)-phenyl]-prop-2-ynyl}-carbamic acid tert-butyl ester

Prepared from 5-(3-bromo-phenyl)-6,8-dimethoxy-7-phenyl-1,3-dihydro-benzo[e][1,4]diazepin-2-one Example 10 according to synthesis of Example 27 and instead of using 3-methoxy-propyne, we used prop-2-ynyl-carbamic acid tert-butyl ester. The title compound (130 mg) was obtained as a brown solid, yield - 56%.
Rf. (AcOEt/CH₂Cl₂ : 4/1) : 0,4
¹H NMR (CDCl₃, 400 MHz): δ 7.57-7.55 (m, 2H), 7.40-7.38 (m, 3H), 7.35-7.32 (m, 4H), 6.64 (s, 1H), 4.84-4.81 and 3.98-3.96 (AB system, J = 11 Hz), 4.74 (large s, 1H), 4.13-4.12 (m, 2H), 3.98 (s, 3H), 3.85 (s, 3H), 3.45 (s, 3H), 2.84 (s, 3H), 1.47 (s, 9H).

### Preparation of Examples of general formula X (Scheme 8)

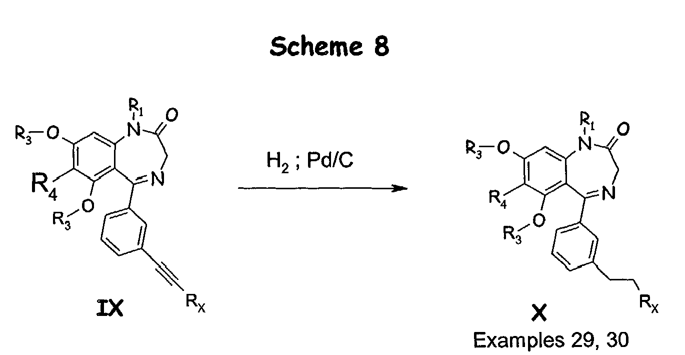

| Examples of general formula X | R₃ | R₄ | R₁ | Rx |
|---|---|---|---|---|
| 29 | OCH₃ | Ph | Me | CH₂OMe |
| 30 | OCH₃ | Ph | Me | CH₂NHBoc |

### Example 29

### 6,8-Dimethoxy-5-[3-(3-methoxy-propyl)-phenyl]-1-methyl-7-phenyl-1,3-dihydro-benzo[e][1,4]diazepin-2-one

A solution of '6,8-dimethoxy-5-[3-(3-methoxy-prop-1-ynyl)-phenyl]-1-methyl-7-phenyl-1,3-dihydro-benzo[e][1,4]diazepin-2-one Example 27 (70 mg, 0.54 mmol) in MeOH/CH₂Cl₂ : 8/2 was degazed with Argon. Then Pd/C 10% (10 mg) was added and the flask was put under Hydrogen for between 6 and 24 hours. The Pd/C was filtered out and washed two times with CH₂Cl₂. The title compound was crystallized from ether/pentane to give a beige solid 45 mg, yield - 64%.
Rf. (AcOEt/CH₂Cl₂ : 4/1) : 0,4
¹H NMR (CDCl₃, 400 MHz): δ 7.40-7.32 (m, 6H), 7.28-7.21 (m, 3H), 6.64 (s, 1H), 4.83-4.80 and 3.99-3.97 (AB system, J = 11 Hz), 3.85 (s, 3H), 3.45 (s, 2H), 3.38-3.35 (m, 2H), 3.31 (s, 3H), 2.82 (s, 3H), 2.70-2.67 (m, 2H), 2.66-2.61 (m, 2H), 1.89-1.85 (m, 2H).

### Example 30

### {3-[3-(6,8-Dimethoxy-1-methyl-2-oxo-7-phenyl-2,3-dihydro-1H-benzo[e][1,4]diazepin-5-yl)-phenyl]-prop-2-ynyl}-carbamic acid tert-butvl ester

Prepared from {3-[3-(6,8-dimethoxy-1-methyl-2-oxo-7-phenyl-2,3-dihydro-1H-benzo[e][1,4]diazepin-5-yl)-phenyl]-prop-2-ynyl}-carbamic acid tert-butyl ester Example 28 according to synthesis of Example 29. The title compound (65 mg) was obtained as a beige solid, yield - 92%.
Rf. (AcOEt/CH₂Cl₂ : 4/1) : 0,3
¹H NMR (CDCl₃, 400 MHz): δ 7.43-7.39 (m, 3H), 7.37-7.27 (m, 3H), 7.26-7.21 (m, 3H), 6.65 (s, 1H), 4.84-4.81 and 3.99-3.97 (AB system, J = 11 Hz), 4.54 (large s, 1H), 3.85 (s, 3H), 3.45 (s, 3H), 3.14-3.12 (m, 2H), 2.83 (s, 3H), 2.66-2.62 (m, 2H), 1.81-1.78 (m, 2H), 1.45 (s, 9H).

### Preparation of Examples of general formula XI and XII (Scheme 9)

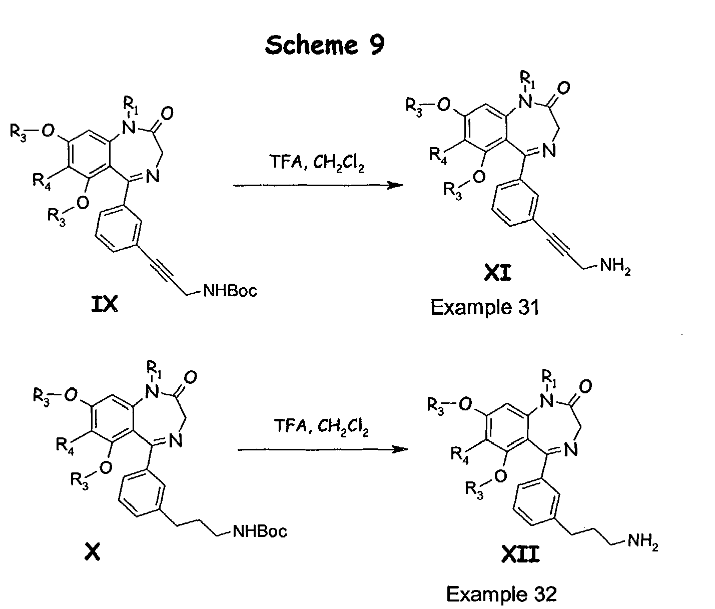

| Examples of general formula I | R₃ | R₄ | R₁ |
|---|---|---|---|
| 31 | OCH₃ | Ph | Me |
| 32 | OCH₃ | Ph | Me |

### Example 31

### 5-[3-(3-Amino-prop-1-ynyl)-phenyl]-6,8-dimethoxy-1-methyl-7-phenyl-1,3-dihydro-benzo[e][1,4]diazepin-2-one

To a solution of '{3-[3-(6,8-dimethoxy-1-methyl-2-oxo-7-phenyl-2,3-dihydro-1H-benzo[e][1,4]diazepin-5-yl)-phenyl]-prop-2-ynyl}-carbamic acid tert-butyl ester Example 28 (55 mg, 0.1 mmol) in 1 mL of degased dichlomethane, at 0°C, were added trifluoroacetic acid (0.5 mL). The mixture was stirred for 1 hours at room temperature under nitrogen atmosphere. The working solution was evaporated under vacuum. The residue was partitioned from a solution of potassium carbonate (10% in water) and ethyl acetate, and extracted two more times with ethyl acetate. The organic phase was dried over Na₂SO₄ and concentrated until dryness. The title compound was crystallised from ether/pentane to afford 35 mg of the title compound: beige solid, yield - 79%.
¹H NMR (CDCl₃, 400 MHz): δ 7.56-7.52 (m, 2H), 7.42-7.38 (m, 3H), 7.35-7.27 (m, 4H), 6.64 (s, 1H), 4.83-4.81 and 3.99-3.96 (AB system, J = 11 Hz), 3.85 (s, 3H), 3.64 (s, 2H), 3.44 (s, 3H), 2.84 (s, 3H).

### Example 32

### 5-[3-(3-Amino-propyl)-phenyl]-6,8-dimethoxy-1-methyl-7-phenyl-1,3-dihydro-benzo[e][1.4]diazepin-2-one

Prepared from {3-[3-(6,8-dimethoxy-1-methyl-2-oxo-7-phenyl-2,3-dihydro-1H-benzo[e][1,4]diazepin-5-yl)-phenyl]-prop-2-ynyl}-carbamic acid tert-butyl ester Example 30 according to synthesis of Example 31. The title compound (25 mg) was obtained as a beige solid, yield - 75%.
¹H NMR (CDCl₃, 400 MHz): δ 7.42-7.40 (m, 2H), 7.39-7.30 (m, 4H), 7.35-7.32 (m, 3H), 6.64 (s, 1H), 4.82-4.79 and 3.99-3.96 (AB system, J = 11 Hz), 3.98 (s, 3H), 3.84 (s, 3H), 3.44 (s, 3H), 2.84 (s, 3H), 2.75-2.72 (m, 2H), 2.68-2.64 (m, 2H), 1.94 (large s, 2H), 1.80-1.78(m, 2H).

### 2. PHARMACOLOGICAL ACTIVITY:

### 2.1. INHIBITION OF PHOSPHODIESTERASES.

### 2.1.1. Isolation of phosphodiesterases from smooth muscle

A 3 g segment of bovine aortic media cut into pieces with scissors was homogenized with an ultra-turrax then a potter glass/glass homogenizer in 7 volumes by weight of buffer A containing a protease inhibitor cocktail (20 mM Tris-HCl, 0.25 M saccharose, 2 mM magnesium acetate, 1 mM dithiothreitol, 5 mM EGTA, 2000 U/ml aprotinin, 10 mg/l leupeptin and 10 mg/l soya trypsic inhibitor). The homogenizate was centrifuged at 105,000 g for 1 hour. The supernatant was loaded on a DEAE-Sephacel column (15 x 1.6 cm) pre-equilibrated with buffer B (buffer A without the saccharose, EGTA and protease inhibitors). The column was washed until there was no detectable absorption at 280 nm, then eluted with a linear gradient of NaCl (0-0.5 M) in buffer B. 3-ml fractions were collected and enzyme activity was determined under the conditions described hereinbelow to localize the different enzymes PDE1, PDE3, PDE4 and PDE5 which were aliquoted and frozen at -80°C (Lugnier et al., Biochem. Phamacol., 1986, 35: 1746-1751). PDE2 was isolated from human platelets, provided by Etablissement Français du Sang-Alsace, according to Kameni Tcheudji JF et al., (J. Mol. Biol. 2001; 310: 181-791), and stored until use at -80°C in small aliquots.

### 2.1.2. Protocol for measuring phosphodiesterase activity

Cyclic nucleotide phosphodiesterase activity was determined by a radioenzymatic method using tritium-labelled cyclic GMP or AMP (1 µM) as substrate (Lugnier et al., 1986). ³H-labelled adenosine or guanosine monophosphate formed by hydrolysis of the radiolabelled cyclic nucleotide was then converted to ³H-labelled adenosine or guanosine in a second reaction with one nucleotidase in excess. The nucleoside formed was separated from the nucleotides by anion exchange chromatography. Nucleoside radioactivity was determined by liquid scintillation counting. Enzymatic incubations were carried out under conditions allowing no more than 15 % hydrolysis of the substrate; each point was performed in duplicate.

### 2.1.3. Determination of inhibition of PDE2.

The concentration of substance which inhibits enzymatic activity by 50 % (IC₅₀) at 1 µM cyclic AMP was calculated by nonlinear regression from the experimental values of hydrolysis rate (Prism, GraphPad).

### 2.1.4. Selectivity

The activity of the compounds was evaluated on other phosphodiesterase isoforms, particularly basal state or calmodulin-activated PDE1 from vascular smooth muscle, PDE3, PDE4 and PDE5 from vascular smooth muscle.

The results obtained are presented in Tables 1 and 2 hereinbelow and are expressed as the percentage inhibition of enzymatic activity produced by 10 µmol of the test compound.

**Table 1**

| Compound represented by formula (I) | | | |
|---|---|---|---|
| Compound | **PDE2 IC₅₀ (µM) or percentage inhibition at 10 µM** | Compound | **PDE2 IC₅₀ (µM) or percentage inhibition at 10 µM** |
| 1 | 9%[3.0] | 17 | 80%[0.8] |
| 2 | 93%[0.73] | 18 | 98%[0.30] |
| 3 | 25% | 19 | 27% |
| 4 | 4% | 20 | 68% |
| 5 | - | 21 | 31% |
| 6 | 80%[1.8] | 22 | 16% |
| 7 | 61%[4.6] | 23 | 15% |
| 8 | 56% | 24 | 18% |
| 9 | 71% | 25 | 18% |
| 10 | 86% | 26 | 5% |
| 11 | 10% | 27 | 83% |
| 12 | 17% | 28 | 43% |
| 13 | 20% | 29 | 91%[0.8] |
| 14 | 9% | 30 | 69% |
| 15 | 9% | 31 | 42% |
| 16 | 18% | 32 | 22% |

**Table 2**

| Selectivity | | | | | |
|---|---|---|---|---|---|
| **Compound** | **IC₅₀ (µM) or percentage inhibition at 10µM** | | | | |
| | **PDE1** | **PDE2** | **PDE3** | **PDE4** | **PDE5** |
| 1 | | 9%[3] | | 43.3% | |
| 2 | | 93%[0.73] | | 58% | |
| 6 | | 80%[1.8] | 40% | 37% | |
| 7 | | 61%[4.6] | | 41% | |
| 17 | 22%[37] | 80%[0.8] | 20%[40] | 23%[47] | 22%[42] |
| 18 | | 98%[0.3] | | 66%[6.5] | |
| 29 | 31% | 91%[0.8] | 40% | 49% | 19% |

All the compounds tested showed potent inhibition of PDE2. The preferred compounds according to the invention have an excellent potency and selectivity profile for phosphodiesterase 2, in so far as said compounds are weaker inhibitors of the other PDEs.

### 2.2. INHIBITION OF 5-HT TRANSPORTER

### 2.2.1 Isolation of 5-HT transporter

The serotonergic 5-HT transporter was isolated from human recombinant HEK-293 cells according to the method described by Tatsumi et al. (1997), Pharmacological profile of antidepressants and related compounds at human monoamine transporters, Eur. J. Pharmacol., 340 : 249-258*.*

### 2.2.2 Protocol for measuring serotonergic transporter binding

Serotonergic transporter binding was determined by a radioligand method using tritium-labelled imipramine (2 nM) as substrate (Tatsumi et al., 1999). Ligand radioactivity was determined by liquid scintillation counting. Ligand incubations were carried out for thirty minutes at 22°C. The experiment was conducted in duplicate.

### 2.2.3. Determination of specific ligand binding

The specific ligand binding to the serotonergic transporter was defined as the difference between the total binding and the non specific binding determined in presence of an excess of unlabelled ligand. The results obtained are presented in Table 3 hereinbelow and are expressed as percent of control specific binding and as a percent inhibition of control specific binding obtained in presence of 10µM of substance.

**Table 3**

| Compound represented by formula (I) | | | |
|---|---|---|---|
| **Compound** | **percentage inhibition at 10 µM** | **Compound** | **percentage inhibition at 10 µM** |
| 1 | 5% | 9 | 1% |
| 2 | 12% | 17 | 98% |
| 7 | 16% | 18 | 0% |
| 8 | 8% | 20 | 16% |

### 2.3. AGONISM OF SIGMA RECEPTORS

### 2.3.1 Isolation of sigma receptors

The sigma receptors were isolated from rat cerebral cortex according to the method described in Shirayama et al., 1993, p-Chlorophenylalanine-reversible reduction of s binding sites by chronic imipramine treatment in rat brain, Eur. J. Pharmacol., 237 : 117-126.

### 2.3.2. Protocol for measuring sigma receptors binding

Sigma 1 receptor binding was determined by a radioligand method using tritium-labelled DTG (8 nM) as substrate (Shirayama et al., 1993). Ligand radioactivity was determined by liquid scintillation counting. For sigma binding evaluation, ligand incubations were carried out for one hundred twenty minutes at 22°C. The experiments were conducted in duplicate.

### 2.3.3. Determination of specific ligand binding

The specific ligand binding to the sigma receptors was defined as the difference between the total binding and the non specific binding determined in presence of an excess of unlabelled ligand. The results obtained are presented in Table 4 hereinbelow and are expressed as percent of control specific binding and as a percent inhibition of control specific binding obtained in presence of 10µM of substance.

**Table 4**

| Compound represented by formula (I) σ (non-selective) | | | |
|---|---|---|---|
| **Compound** | **percentage inhibition at 0.1 µM** | **percentage inhibition at 1 µM** | **percentage inhibition at 10 µM** |
| 17 | 1% | 57% | 100% |

### 2.3.4. Isolation of sigma 1 and 2 receptors

The sigma 1 receptors were isolated from guinea pig cerebral cortex according to the method described in Bowen et al., 1993. The sigma 2 receptors were isolated from rat cerebral cortex according to the method described in Bowen et al., 1993_( BOWEN, W.D., de COSTA, B.R., HELLEWELL, S.B., WALKER, M. and RICE, K.C. (1993) [3H]-(+)-pentazocine : a potent and highly selective benzomorphan-based probe for sigma, receptors. Mol. Neuropharmacol., 3 : 117-126)

### 2.3.5. Protocol for measuring sigma receptors binding

Sigma 1 receptor binding was determined by a radioligand method using tritium-labelled (+)pentazocine (2 nM) as substrate (Bowen et al., 1993). Sigma 2 receptor binding was determined by a radioligand method using tritium-labelled DTG (+300 nM (+)pentazocine) (5 nM) as substrate (Bowen et al., 1993). Ligand radioactivity was determined by liquid scintillation counting. For sigma 1 binding evaluation, ligand incubations were carried out for one hundred fifty minutes at 22°C. For sigma 2 binding evaluation, ligand incubations were carried out for one hundred twenty minutes at 22°C The experiments were conducted in duplicate.

### 2.3.6. Determination of specific ligand binding

The specific ligand binding to the sigma receptors was defined as the difference between the total binding and the non specific binding determined in presence of an excess of unlabelled ligand. The results obtained are presented in Table 4 hereinbelow and are expressed as percent of control specific binding and as a percent inhibition of control specific binding obtained in presence of 10µM of substance.

**Table 5**

| Compound represented by formula (I) σ (selective) | | |
|---|---|---|
| **Compound** | **percentage inhibition at 10 µM** | |
| | **σ₁** | **σ₂** |
| 17 | 93% | 93% |

### 2.4. IN VITRO PHARMACOLOGY :σ RECEPTORS

### 2.4.1. Isolated Organ Bioassay

### General Procedures

| **Assay** | **Tissue** | **Reference agonist** | **Response** | **Reference antagonist** | **Bibliography** |
|---|---|---|---|---|---|
| σ | guinea pig vas deferens (field-stimulated) | (+)SKF 10,047 | enhancement of twitch contraction | rimcazole | Vaupel and Su (1987) |

VAUPEL, D.B. and SU, T.P. (1987), Guinea-pig vas deferens preparation may contain both σ and phencyclidine receptors, Eur. J. Pharmacol., 139 : 125-128.

### Experimental Conditions

Segments of guinea pig vas deferens were suspended in 20-ml organ baths containing an oxygenated (95 % O₂ and 5 % CO₂) and pre-warmed (37°C) physiological salt solution of the following composition (in mM): NaCl 118.0, KCl 4.7, MgSO₄ 1.2, CaCl₂ 2.5, KH₂PO₄ 1.2, NaHCO₃ 25.0 and glucose 11.0 (pH 7.4).
Yohimbine (1 µM), (-)sulpiride (1 µM), atropine (1 µM) and naloxone (1 µM) were also present throughout the experiments to block the α₂-adrenergic, dopamine D2, muscarinic and opioid receptors, respectively.

The tissues were connected to force transducers for isometric tension recordings. They were stretched to a resting tension of 0.5 g, allowed to equilibrate for at least 30 min during which time they were washed repeatedly and the tension readjusted. Thereafter, they were stimulated electrically using a constant current stimulator.
The experiments were carried out using a semi-automated isolated organ system possessing eight organ baths, with multichannel data acquisition.

### 2.4.2. Experimental Protocols

### Test for agonist activity

The tissues were exposed to a submaximal concentration of the reference agonist (+)SKF-10047 (100 µM) to verify responsiveness and to obtain a control response.
Following washings and recovery of the twitch contraction amplitude, the tissues were exposed to increasing concentrations of substance or the same agonist. The different concentrations were added cumulatively and each was left in contact with the tissues until a stable response was obtained or for a maximum of 15 min.
If an agonist-like response (enhancement of twitch contractions) was obtained, the reference antagonist rimcazole (10 µM) was tested against the highest concentration of substance to confirm the involvement of the σ receptors in this response.

### Test for antagonist activity

The tissues were exposed to a submaximal concentration of the reference agonist (+)SKF-10047 (100 µM) to obtain a control response.
After stabilization of the (+)SKF-10047-induced response, increasing concentrations of substance or the reference antagonist rimcazole were added cumulatively. Each concentration was left in contact with the tissues until a stable response was obtained or for a maximum of 15 min.
If it occurred, an inhibition of the (+)SKF-10047-induced increase in twitch contraction amplitude by **substance** indicated an antagonist activity at the σ receptors.

### 2.4.3. Analysis and Expression of Results

The parameter measured was the maximum change in the twitch contraction amplitude induced by each compound concentration.
The results are expressed as a percent variation of the control twitch contraction amplitude (mean values).

The effects of substance tested at 1.0 10⁻⁶ M, 3.0 10⁻⁶ M and 1.0 10⁻⁵ M for agonist and antagonist activities at the σ receptors in the guinea pig vas deferens bioassay are summarized in Tables 6-7 where those of the reference compounds are also reported.

### Tables 6 - 7

### Effects of compound 17 investigated for agonist and antagonist activities at the σ receptors in the guinea pig vas deferens

### Test for agonist activity

| Compounds | Control response to (+)SKF-10,047 (1.0 10⁻⁴ M) | Responses to increasing concentrations of the compounds | | | + Rimcazole (1.0 10⁻⁵ M) |
|---|---|---|---|---|---|
| | | 1.0 10⁻⁶M | 3.0 10⁻⁶M | 1.0 10⁻⁵ M | |
| **17** | **+126** | **0** | **0** | **+33** | **-9** |
| | | 1.0 10⁻⁵ M | 3.0 10⁻⁵ M | 1.0 10⁻⁴M | |
| **(+)SKF-10,047** | **+ 155** | **+ 40** | **+ 90** | **+ 158** | **-8** |

### Test for antagonist activity

| Compounds | Control response to (+)SKF-10,047 (1.0 10⁻⁴ M) | Responses to (+)SKF-10,047 (1.0 10⁻⁴ M) in the presence to increasing concentrations of the compounds | | |
|---|---|---|---|---|
| | | 1.0 10⁻⁶M | 3.0 10⁻⁶M | 1.0 10⁻⁵ M |
| **17** | **+ 139** | **+ 139** | **+139** | **+ 169** |
| | | 1.0 10⁻⁶M | 3.0 10⁻⁶M | 1.0 10⁻⁶ M |
| **rimcazole** | **+ 142** | **+ 111** | **+ 52** | **- 12** |

| | | | | |
|---|---|---|---|---|
| The results are expressed as a percent variation of the control twitch contraction amplitude (mean values; n=2). The signs + and - indicate an increase and a decrease, respectively. | | | | |

In the field-stimulated guinea pig vas deferens, the σ receptor agonist (+)SKF 10,047 induced a concentration-dependent enhancement of the twitch contraction amplitude, which was inhibited by the antagonist rimcazole.
In the untreated tissues, subtance was inactive at 1.0 10⁻⁶ M and 3.0 10⁻⁶ M. At 1.0 10⁻⁵ M, it caused a rimcazole-sensitive enhancement of the twitch contraction amplitude.

In the tissues previously exposed to (+)SKF 10,047, substance did not affect the response to this agonist at 1.0 10⁻⁶ M and 3.0 10⁻⁶ M. At 1.0 10⁻⁵ M, it caused a further enhancement of the twitch contraction amplitude.
These results indicate that in this tissue the compound behaves as an agonist at the σ receptors at concentrations higher than 3.0 10⁻⁶ M.

### 3. BEHAVIOURAL TESTS

### SWIM TEST

This test is based on the induction of alternative behaviour in rodents subjected to an acute stress. In this model, the rat or mouse placed in a container filled with water show periods of increased swimming activity and periods of relative immobility. Clinically active anti-depressants have been found to delay the onset of the first phase of immobility and to reduce the total time of relative immobility.

Swiss mice were used. The animal was placed individually in the water where it remained for 6 minutes. The animal was given an accommodation period of 2 minutes. During the last 4 minutes observation period, the onset of the first period of immobility and the duration of the periods of immobility were recorded.

Treatment was administered 60 minutes prior the test. Animals were randomly distributed in 4 groups. Control group received the vehicle whereas the other 3 groups received different single dose of test compound.

Results are illustrated in figures 1a and 1b : Mean Duration of Phases of Immobility (s) ; N= 10; p<0,005 (Dunnett's test).

Results are given by figures 1a (onset of time of immobility) and 1b (total immobility time). Statistical analyses revealed a significant difference between groups regarding the period of total immobility (p = 0.005). Mice treated with 0.3, 3 or 30 mg/kg of test compound showed significantly shorter time of relative immobility than control animals.

### LIGHT DARK TEST

### 1. Purpose

The light dark (LD) test is used to evaluate the relative anxiety status of mice.

### 2. Background

The light dark paradigm in rodents is based on a conflict between the innate aversion to brightly illuminated areas and the spontaneous exploratory activity. If given a choice between a large brightly compartment versus a small dark compartment, animals spontaneously prefer the dark. Anxiolytic compounds have been found to increase the number of entries into the bright compartment and the total duration of time spent there. Anxiogenic compounds were observed to work in the opposite way.

### 3. Materials

### Equipment

The apparatus consists of two polyvinylchloride boxes (19 x 19 x 15 cm) closed with plexiglas. One of these boxes is illuminated by a 100 W desk lamp placed 15 cm above and providing an illumination of about 4400 Lux, the other box being dark. An opaque plastic tunnel (5 x 7 x 10 cm) separates the dark box from the illuminated one.

### 4. Methods

### Step 1 - drug treatments:

Animals are randomly assigned to test compounds of the invention (test substances) and control groups. Each animal is treated with vehicle or test compounds one hour before the test at appropriate doses and using the oral route of administration.

### Step 2 - test implementation:

The animal is placed in the lit box, with the head directed towards the tunnel. The time spent in the lit box are recorded over a 5 minutes period after the first entry of the animal in the dark box.

The apparatus is cleaned between each animal using alcohol (70°).

### 5. Data analysis and results

All animals scored without entry into the lit box are excluded from the analysis.

A one-way analysis of variance (ANOVA) is used to test whether the mean of the number of entries into lit box or the mean of the time spent in the lit box differs among three or more groups. Where ANOVA indicates a significant difference (p ≤ 0.05), Fisher's Protected Least Significant Difference is used to compare pairs of group means.
Results are shown in figure 1 c.

## Claims

1. A compound of general formula (I): 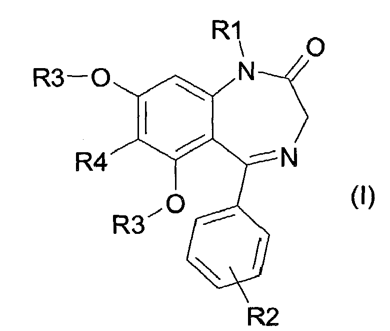 wherein:
. R₁ represents an hydrogen atom, an alkyl, aryl, alkylaryl, or arylalkyl group, wherein said group can be substituted by at least one group preferably selected among the following groups : alkyl group, a halogen atom, a halogenoalkyl group, such as trifluoromethyl or difluoromethyl group,
. R₂ represents a hydrogen atom, a halogen atom, an alkyl, an alkoxy, an alkoxyalkyl, an alkoxyalkynyl, an aminoalkyl, a trifluoromethyl, an alkenyl, an alkynyl, an aminoalkynyl, a hydroxy group, CN, CHO, CONH₂ group, or a group of the following formula: (R₅)ₙNHCOR₆, where R₅ is an alkyl, alkenyl or alkynyl group , n is 0 to 3, R₆ is an alkyl, aryl, aryloxy or alkoxy group,
. R₃, which is the same or different, is a hydrogen atom, an alkyl, or a halogenoalkyl group, such as for instance trifluoromethyl or difluoromethyl,
. R₄ represents an aryl or heteroaryl group, said aryl or heteroaryl group may be substituted by at least one group preferably selected among the following groups: a halogen atom, an alkyl, alkoxy, a halogenoalkyl group, such as for instance trifluoromethyl or difluoromethyl,
or a pharmaceutically acceptable salt or solvate thereof.

2. A compound according to claim 1, wherein R₁ represents a hydrogen atom or an alkyl group, preferably a hydrogen atom, a methyl, ethyl, propyl, or cyclopropylmethyl group.

3. A compound of formula (I) according to claim 1, wherein R₁ represents an arylalkyl group, in particular a phenylalkyl group, such as benzyl, phenethyl or 3-phenyl-propyl, in which the aryl group may be substituted by a halogenoalkyl group.

4. A compound of formula (I) according to any one of the preceding claims, wherein R₂, which is different from hydrogen, is on position 3 of the phenyl group.

5. A compound of formula (I) according to any one of claims 1-4, wherein R₂ represents a hydrogen atom, a halogen atom (preferably Br), CN, or CONH2.

6. A compound according to any one of claims 1-4, wherein R₂, represents 3-alkoxypropynyl (preferably 3-methoxypropynyl), 3-aminopropynyl, 3-alkoxypropyl (preferably 3-methoxypropyl), or 3-aminopropyl.

7. A compound according to any one of claims 1-4, wherein R₂ represents a group of the following formula: -(R₅)ₙNHCOR₆ wherein R₅ is an alkyl (preferably propyl) or alkynyl (preferably propynyl) group, n is 1 to 3, and R₆ is an alkoxy (preferably tert-butoxy) group.

8. A compound of formula (I) according to any one of the preceding claims, wherein R₃, which is the same or different, represents an alkyl group, preferably a methyl group.

9. A compounds of formula (I) according to any one of the preceding claims, wherein R₄ is a, substituted or not, phenyl group.

10. A compound of formula (I) according to any one of the preceding claims 1-8, wherein R₄ is a, substituted or not, pyridine group.

11. A compound according to any one of the preceding claims 1-10, wherein it is selected in the group consisting of:
3-(6,8-Dimethoxy-2-oxo-7-phenyl-2,3-dihydro-1H-benzo[e][1,4]diazepin-5-yl)-benzonitrile
3-[7-(4-Chloro-phenyl)-6,8-dimethoxy-2-oxo-2,3-dihydro-1H-benzo[e][1,4]diazepin-5-yl]-benzonitrile
6,8-Dimethoxy-5,7-diphenyl-1,3-dihydro-benzo[e][1,4]diazepin-2-one
3-(6,8-Dimethoxy-2-oxo-7-pyridin-3-yl-2,3-dihydro-1H-benzo[e][1,4]diazepin-5-yl)-benzonitrile
5-(3-Bromo-phenyl)-6,8-dimethoxy-7-phenyl-1,3-dihydro-benzo[e][1,4]diazepin-2-one
3-(6,8-Dimethoxy-1-methyl-2-oxo-7-phenyl-2,3-dihydro-1H-benzo[e][1,4]diazepin-5-yl)-benzonitrile
3-[7-(4-Chloro-phenyl)-6,8-dimethoxy-1-methyl-2-oxo-2,3-dihydro-1H-benzo[e][1,4]diazepin-5-yl]-benzonitrile
3-(6,8-Dimethoxy-1-methyl-2-oxo-7-pyridin-3-yl-2,3-dihydro-1H-benzo[e][1,4]diazepin-5-yl)-benzonitrile
6,8-Dimethoxy-1-methyl-5,7-diphenyl-1,3-dihydro-benzo[e][1,4]diazepin-2-one
5-(3-Bromo-phenyl)-6,8-dimethoxy-1-methyl-7-phenyl-1,3-dihydro-benzo[e][1,4]diazepin-2-one
3-(6,8-Dimethoxy-2-oxo-7-phenyl-1-ethyl-2,3-dihydro-1H-benzo[e][1,4]diazepin-5-yl)-benzonitrile
3-(6,8-Dimethoxy-2-oxo-7-phenyl-1-propyl-2,3-dihydro-1H-benzo[e][1,4]diazepin-5-yl)-benzonitrile
3-(1-Cyclopropylmethyl-6,8-dimethoxy-2-oxo-7-phenyl-2,3-dihydro-1H-benzo[e][1,4]diazepin-5-yl)-benzonitrile
3-(1-Benzyl-6,8-dimethoxy-2-oxo-7-phenyl-2,3-dihydro-1H-benzo[e][1,4]diazepin-5-yl)-benzonitrile
3-[6,8-Dimethoxy-2-oxo-7-phenyl-1-(4-trifluoromethyl-benzyl)-2,3-dihydro-1H-benzo [e][1,4]diazepin-5-yl]-benzonitrile
3-[6,8-Dimethoxy-2-oxo-7-phenyl-1-(3-phenyl-propyl)-2,3-dihydro-1H-benzo[e][1,4]diazepin-5-yl]-benzonitrile
3-(6,8-Dimethoxy-1-methyl-2-oxo-7-phenyl-2,3-dihydro-1H-benzo[e][1,4]diazepin-5-yl)-benzamide
3-[7-(4-Chloro-phenyl)-6,8-dimethoxy-1-methyl-2-oxo-2,3-dihydro-1H-benzo[e][1,4]diazepin-5-yl]-benzamide
3-(6,8-Dimethoxy-2-oxo-7-pyridin-3-yl-2,3-dihydro-1H-benzo[e][1,4]diazepin-5-yl)-benzamide
3-(6,8-Dimethoxy-2-oxo-7-pyridin-3-yl-2,3-dihydro-1H-benzo[e][1,4]diazepin-5-yl)-benzamide
3-(6,8-Dimethoxy-2-oxo-7-phenyl-1-ethyl-2,3-dihydro-1H-benzo[e][1,4]diazepin-5-yl)-benzamide
3-(6,8-Dimethoxy-2-oxo-7-phenyl-1-propyl-2,3-dihydro-1H-benzo[e][1,4]diazepin-5-yl)-benzamide
3-(1-Cyclopropylmethyl-6,8-dimethoxy-2-oxo-7-phenyl-2,3-dihydro-1H-benzo[e][1,4]diazepin-5-yl)-benzamide
3-(1-Benzyl-6,8-dimethoxy-2-oxo-7-phenyl-2,3-dihydro-1H-benzo[e][1,4]diazepin-5-yl)-benzamide
3-[6,8-Dimethoxy-2-oxo-7-phenyl-1-(4-trifluoromethyl-benzyl)-2,3-dihydro-1H-benzo[e][1,4]diazepin-5-yl]-benzamide
3-[6,8-Dimethoxy-2-oxo-7-phenyl-1-(3-phenyl-propyl)-2,3-dihydro-1H-benzo[e][1,4]diazepin-5-yl]-benzamide
6,8-Dimethoxy-5-[3-(3-methoxy-prop-1-ynyl)-phenyl]-1-methyl-7-phenyl-1,3-dihydro-benzo[e][1,4]diazepin-2-one
{3-[3-(6,8-Dimethoxy-1-methyl-2-oxo-7-phenyl-2,3-dihydro-1H-benzo[e][1,4]diazepin-5-yl)-phenyl]-prop-2-ynyl}-carbamic acid tert-butyl ester
6,8-Dimethoxy-5-[3-(3-methoxy-propyl)-phenyl]-1-methyl-7-phenyl-1,3-dihydro-benzo[e][1,4]diazepin-2-one
{3-[3-(6,8-Dimethoxy-1-methyl-2-oxo-7-phenyl-2,3-dihydro-1H-benzo[e][1,4]diazepin-5-yl)-phenyl]-prop-2-ynyl}-carbamic acid tert-butyl ester
5-[3-(3-Amino-prop-1-ynyl)-phenyl]-6,8-dimethoxy-1-methyl-7-phenyl-1,3-dihydro-benzo[e][1,4]diazepin-2-one
5-[3-(3-Amino-propyl)-phenyl]-6,8-dimethoxy-1-methyl-7-phenyl-1,3-dihydro-benzo[e][1,4]diazepin-2-one.

12. A pharmaceutical composition comprising at least one compound of formula (I), as defined in any one of the preceding claims, and a pharmaceutically acceptable vehicle or support.

13. The pharmaceutical composition according to claim 12, for the treatment of diseases associated with abnormal regulation of intracellular cAMP and/or cGMP rate.

14. The use of a compound of any one of the claims 1-11 for the preparation of a pharmaceutical composition for the treatment of diseases of the central nervous system, especially connected with an abnormal regulation of neurotransmitter effect or a release deficiency of one of the neurotransmitters.

15. The use of a compound of any one of the claims 1-11 for the preparation of a pharmaceutical composition for the treatment of a disease selected in the group consisting of depression, schizophrenia, autism, anxiety, bipolar disorder, attention deficit hyperactivity disorder (ADHD), convulsion, sleeping disorders, obsessive compulsive disorders (OCD), Post Traumatic Stress Disorder (PTSD), fibromyalgia, Tourette's syndrome, drug or alcohol dependence, epilepsia, movement disorders, such as dystonia and tardive dyskinesia, Alzheimer's disease, Huntington's chorea, Parkinson's disease, amyotrophic lateral sclerosis, multiple sclerosis, obesity, Restless Legs Syndrome (RLS), psychosis, cerebrovascular diseases, migraine, convulsion, amnesia, premenstrual dysphoric disorder (PMDD), post-traumatic stress disorder (PTSD), panic disorders, memory deficiency, cognitive disorders, social disorders, bulimia nervosa, dementia (in particular Lewy body dementia or senile dementia of the Alzheimer type), rheumatism, sepsis, diabetes-induced pathologies, cancer, autoinflammatory diseases, dysfunction of liver due to ageing, disorders due to Trypanosoma (such as sleeping sickness and nagana) and Candida albicans.
